# EUROPEAN PATENT APPLICATION

(11) **EP 4 432 295 A1**
(43) Date of publication of application: **18.09.2024**
(21) Application number: 24162927.8
(22) Date of filing: 12.03.2024
(51) Int. Cl.: G16H 20/17, G06K 19/00, G16H 40/63, G16H 40/67

(54) **WIRELESS COMMUNICATION TAGS TO ENHANCE AID SYSTEM PERFORMANCE**

(30) Priority: 13.03.2023 US 202363489864 P
(71) Applicant: Insulet Corporation, Acton MA 01720 (US)
(72) Inventor: NARAYANASWAMI, Rangarajan, Weston, MA, 02493 (US)
(74) Representative: Peterreins Schley

(57) **Abstract**

A system for utilizing wireless communication tags with wearable medical devices is presented. The system includes a user device. The system includes at least a wireless communication tag. The at least a wireless communication tag is configured to communicate physiology affecting information to the user device. The user device is configured to modify a mode of operation of a wearable medical device, such as a wearable injection device, as a function of the physiology affecting information.

## Description

### TECHNICAL FIELD

The present subject matter generally relates to wireless communication tags. In particular, the present subject matter relates to a system in which wireless communication tags are operable to improve the operation of an automated insulin delivery system for wearable medical devices.

### BACKGROUND

Patients with diabetes may be challenged with estimating carbohydrate content in meals. This is especially true for young children as well as the elderly. Challenges may also arise when the meal is low in carbohydrates, but high in protein/fat. Further, restaurant meals may be hard to estimate for all age groups. These factors may contribute to incorrect bolusing as well as increased patient burden.

### SUMMARY OF THE DISCLOSURE

In an aspect, a system of wireless communication tags for wearable medical devices is presented. The system includes a user device and a wireless communication tag. The wireless communication tag may be configured to communicate a physiology affecting information to the user device. The user device is configured to modify a mode of operation of a wearable medical device as a function of the physiology affecting information.

In an aspect, a wireless communication tag is presented. The wireless communication tag includes at least a processor and a transmission element in communication with the processor. The wireless communication tag includes a memory communicatively connected to the processor. The memory contains instructions configuring the processor to establish a data connection with at least a user device through the transmission element. The processor is configured to communicate a physiology affecting information to the user device through the data connection.

These and other aspects and features of non-limiting embodiments of the present subject matter will become apparent to those skilled in the art upon review of the following description of specific non-limiting embodiments of the subject matter in conjunction with the accompanying drawings.

### BRIEF DESCRIPTION OF THE DRAWINGS

FIG. 1 is an exemplary embodiment of a system incorporating wireless communication tags with wearable medical devices;
FIG. 2 is an exemplary embodiment of a wearable medical device;
FIG. 3 is an exemplary embodiment of a wireless communication tag;
FIG. 4A is an exemplary embodiment of a meal identification system for wearable medical devices;
FIG. 4B illustrates an exemplary implementation of a wireless communication tag with meal container;
FIG. 4C illustrates an exemplary process utilizing the wireless communication tag and meal container shown in FIG. 4B;
FIG. 5 is an exemplary embodiment of an exercise identification system useable with wearable medical devices;
FIG. 6 is an exemplary embodiment of a driving identification system usable with wearable medical devices;
FIG. 7 is an exemplary embodiment of a sleeping identifying system usable with wearable medical devices; and
FIG. 8 is an exemplary embodiment of a system for controlling administration of a drug treatment plan utilizing wireless communication tags incorporated in wearable medical devices.

### DETAILED DESCRIPTION

In the following description, for the purposes of explanation, numerous specific details are set forth in order to provide a thorough understanding of the present subject matter. It will be apparent, however, that the present subject matter may be practiced without these specific details. As used herein, the word "exemplary" or "illustrative" means "serving as an example, instance, or illustration." Any implementation described herein as "exemplary" or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims.

At a high level, aspects of the present disclosure are related to systems for wireless communication tags (e.g., smart tags) for drug delivery devices (e.g. wearable medical devices). Aspects of the present disclosure may be used to modify modes of operation of one or more drug delivery devices, particularly one or more wearable medical devices. Modifying the mode of operation may include adjusting one or more parameters of the drug delivery device. Adjusting one or more parameters of the drug delivery device may for instance include one or more of, adjusting a target blood glucose level of the user, adjusting a drug delivery schedule, adjusting a frequency of blood glucose sensing and/or switching to another predetermined mode of operation or generating a mode of operation. In some embodiments, aspects of the present disclosure may be used to provide accurate meal data to a user device which may be used for a mode of operation for a wearable medical device. In another embodiment, aspects of the present disclosure may be used to provide smart environments for wearable medical devices.

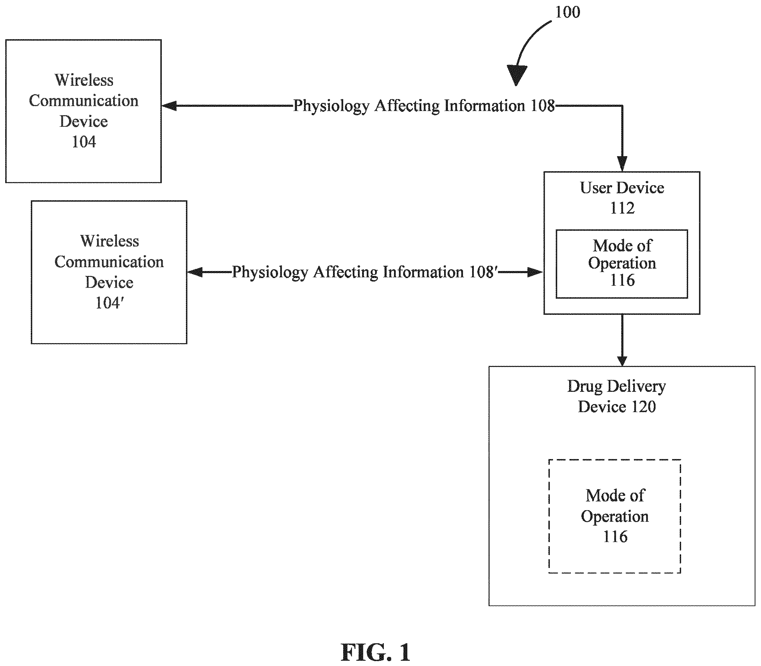

FIG. 1 is an exemplary embodiment of a system for smart tags usable with wearable medical devices. The example system 100 may include a number of different wireless communication tags, such as 104 and 104'. For ease of discussion, only the two wireless communication tags 104 and 104 ' are discussed in detail, but there may be tens or hundreds of different wireless communication tags. A "wireless communication tag" as used in this disclosure is a device capable of wireless transmission of data. Wireless communication tag 104 may include one or more conductive elements such as, but not limited to, copper, aluminum, and/or other metals. Conductive elements of wireless communication tag 104 may be shaped in one or more coils. Coils of conductive elements may be receptive to and/or generate one or more magnetic fields. The wireless communication tag 104 may include one or more coils of one or more conductive elements that may be responsive to changes in magnetic fields. For instance, and without limitation, the wireless communication tag 104 may include a single coil of conductive element encased in a substrate. Substrates may include, but are not limited to, epoxy, plastic, and/or other substrates. The wireless communication tag 104 may include an NFC sticker. An NFC sticker may include one or more coils of conductive material encased in a substrate having an adhesive side.

Still referring to FIG. 1, wireless communication tag 104 may include, but is not limited to, Bluetooth^{®} devices, Wi-Fi devices, local area network (LAN) devices, and the like. In some embodiments, wireless communication tag 104 may include a smart tag. A "smart tag" as used in this disclosure is a portable device capable of sending and/or receiving data wirelessly. Examples of data that may be sent by a smart tag may include location information, environment (e.g., humidity, temperature and the like) information, context information (e.g., food composition, tag identifier, etc.) and the like. Smart tags may include, but are not limited to, radio frequency identification (RFID) devices, near field communication (NFC) devices, Bluetooth^{®} devices, Wi-Fi devices, and the like. Smart tags may include, but are not limited to, Apple AirTags^{®}, Galaxy SmartTags^{®}, Tile^{®} smart tags, and the like.

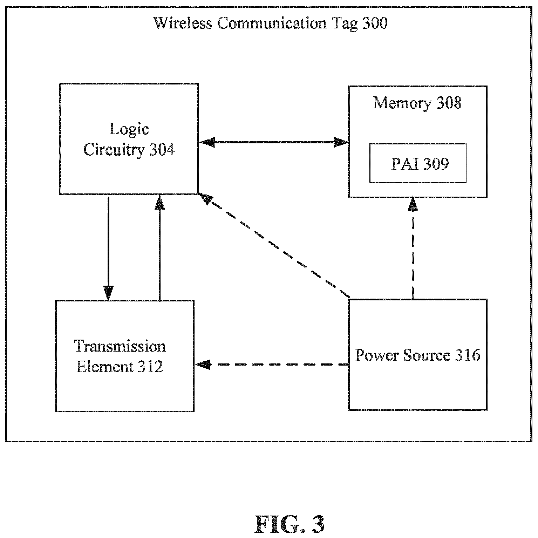

Still referring to FIG. 1, in some embodiments, wireless communication tag 104 may be passively powered. "Passively powered" as used in this disclosure is a form of power that relies on external power sources. Passive power systems may include, without limitation, passive RFID and/or NFC systems, piezo-electric devices and the like. In some embodiments, wireless communication tag 104 may be actively powered. "Actively powered" as used in this disclosure is a form of power that relies on internal power sources. Active power systems may include, without limitation, one or more batteries, capacitors, transistors, and the like. Wireless communication tag 104 may be described in further detail below with reference to FIG. 3.

Still referring to FIG. 1, in some embodiments, the system 100 may include user device 112. A "user device" as used in this disclosure is a computing device operated by one or more individuals. User device 112 may include, but is not limited to, smartphones, laptops, desktops, tablets, personal diabetes management (PDM) devices, and the like. User device 112 may include any computing device as described in this disclosure, including without limitation a microcontroller, microprocessor, digital signal processor (DSP) and/or system on a chip (SoC) as described in this disclosure. User device 112 may include, be included in, and/or communicate with a mobile device such as a mobile telephone or smartphone. The user device 112 may include a single computing device operating independently, or may include two or more computing device operating in concert, in parallel, sequentially or the like; two or more computing devices may be included together in a single computing device or in two or more computing devices. The user device 112 may interface or communicate with one or more additional devices as described below in further detail via a network interface device. In an example, a network interface device (not shown) may be utilized for connecting user device 112 to one or more of a variety of networks, and one or more devices. Examples of a network interface device include, but are not limited to, a network interface card (e.g., a mobile network interface card, a LAN card), a modem, and any combination thereof. Examples of a network include, but are not limited to, a wide area network (e.g., the Internet, an enterprise network), a local area network (e.g., a network associated with an office, a building, a campus or other relatively small geographic space), a cellular network, a data network associated with a cellular provider, a direct connection between two computing devices, and any combinations thereof. A network may employ a wired and/or a wireless mode of communication. In general, any network topology may be used. Information (e.g., data, software etc.) may be communicated to and/or from a computer and/or a computing device.

With continued reference to FIG. 1, user device 112, and/or any other computing device as described throughout this disclosure, may be designed and/or configured to perform any method, method step, or sequence of method steps in any embodiment described in this disclosure, in any order and with any degree of repetition. For instance, user device 112 may be configured to perform a single step or sequence repeatedly until a desired or commanded outcome is achieved; repetition of a step or a sequence of steps may be performed iteratively and/or recursively using outputs of previous repetitions as inputs to subsequent repetitions, aggregating inputs and/or outputs of repetitions to produce an aggregate result, reduction or decrement of one or more variables such as global variables, and/or division of a larger processing task into a set of iteratively addressed smaller processing tasks. User device 112 may perform any step or sequence of steps as described in this disclosure in parallel, such as simultaneously and/or substantially simultaneously performing a step two or more times using two or more parallel threads, processor cores, or the like; division of tasks between parallel threads and/or processes may be performed according to any protocol suitable for division of tasks between iterations. Persons skilled in the art, upon reviewing the entirety of this disclosure, will be aware of various ways in which steps, sequences of steps, processing tasks, and/or data may be subdivided, shared, or otherwise dealt with using iteration, recursion, and/or parallel processing.

Still referring to FIG. 1, in some embodiments, wireless communication tag 104 may be configured to transmit physiology affecting information 108. Wireless communication tag 104 may be operable to transmit a physiology affecting information through, without limitation, one or more frequencies of electromagnetic radiation. Frequencies of electromagnetic radiation may include, but are not limited to, 2.4 GHz, 5GHz, 10GHz, and the like. In other words, the wireless communication tag 104 may transmit data, such as physiology affecting information 108, through one or more of Bluetooth^{®}, Wi-Fi, and/or other wireless communication signals. "Physiology affecting information" as used in this disclosure is data pertaining to activities or items that may cause a potential change in a user's physiology. The term "physiology" as used in this disclosure is the biological composition of an individual, such as blood glucose levels, ketone levels, blood oxygen levels, protein levels, hormone levels, and the like. Items that may cause a potential change in a user's physiology may include, for example, food and drinks (e.g., carbohydrates), medications, exercise, ambient temperature and humidity, and the like. Potential changes in a user's activity may also affect the user's physiology and may include, without limitation, eating, sleeping, exercising, and the like. In some embodiments, potential changes in a user's activity may also affect the user's physiology. The user's activity may include the activity of operating machinery, such as cars, trucks, motorcycles, exercise machines, and the like. For example, a person's physiology may change due to the stress induced by operating machinery (e.g., operating a vehicle in dense traffic or at a high speed, such as highway speeds), release of hormones in response to the activity, or the like.

Still referring to FIG. 1, physiology affecting information 108 may include meal data related to the nutritional composition of a meal. "Meal data" as used in this disclosure may be information pertaining to one or more comestible items. Meal data may include types of foods. Types of foods may, for example, include meats, poultry, dairy products, vegetables, fruits, juices, seafood, and the like. Meal data may include a quantity of one or more foods and/or food types. As a non-limiting example, meal data may include a quantity of 2 chicken breasts, 2 slices of whole wheat bread, and 12 oz of fat free milk. In some embodiments, meal data may include macronutrient amounts. A "macronutrient" as used in this disclosure is a protein, a fat, and/or carbohydrates (also referred to as "carbs"). Meal data may include amounts of macronutrients in grams or other units of measurements. For instance, and without limitation, meal data may include macronutrient amounts of 20 g of protein, 8 g of fat, and 10 g of carbs. In some embodiments, meal data may include micronutrient amounts. "Micronutrients" as used in this disclosure are vitamins and/or minerals. Meal data may include micronutrient data such as, but not limited to, iron, vitamin A, vitamin B, vitamin C, vitamin D, vitamin K, iodine, folate, zinc, and the like. In some embodiments, meal data may include one or more food additives. A "food additive" as used in this disclosure is an element added to one or more food items. Food additives may include without limitation, preservatives, sweeteners, flavorings, and the like. The meal data may, for example, include a combination of macronutrients, micronutrients, and/or food additives. In some embodiments, meal data may include a meal type. A "meal type" as used in this disclosure is a category of one or more food items. Meal types may include, without limitation, breakfast, lunch, dinner, snacks, pre-workout meals, post-workout meals, and the like. As a non-limiting example, meal data may include a breakfast of 3 egg whites, 8 oz. of orange juice, and two multi-grain toast slices, having a macronutrient breakdown of 20 grams of protein, 34 grams of carbs, and 8 grams of non-saturated fat.

The physiology affecting information 108 may also be exercise data. "Exercise data" as used in this disclosure is information relating to exertion of a user's body, the location of where the user exercises, a type of exercise equipment used by the user, or the like. Exercise data may be an exercise type. An "exercise type" as used in this disclosure is a category of physical exertion. For example, exercise types may include, without limitation, stretches, warm ups, aerobic exercise, anaerobic exercise, endurance training, strength training, and the like. The exercise data may also include a duration of exercise, such as, without limitation, seconds, minutes, hours, and/or any combination thereof. Alternatively, or additional, the exercise data may include a time stamp from which the duration of the activity (i.e., exercise) may be calculated. The exercise data may also include a number of sets of an exercise type. For instance, 3 sets of barbell squats or the like. In some embodiments, exercise data may include a number of repetitions, such as 1, 5, 10, and the like, without limitation. Exercise data may include data on one or more muscles targeted, such as, without limitation, arms, legs, chest, back, core, and the like. For instance, exercise data may include a stationary bike ride on a level 7 out of 10 resistance level with a duration of 30 mins and an average heart rate of 150 beats per minute (bpm).

Still referring to FIG. 1, in some embodiments, physiology affecting information 108 may include driving data that is related to the activity of driving a vehicle. For example, "driving data" as used in this disclosure is information pertaining to the activity of a user operating a vehicle. Operation of a vehicle may include interacting with one or more control interfaces of a vehicle, such as, but not limited to, wheels, brakes, pedals, gear shifters, and the like. Driving data may include one or more vehicle types. Vehicle types may include, but are not limited to, cars, trucks, motorcycles, bicycles, scooters, planes, boats, and the like. Driving data may include a duration of a driving of one or more vehicles, such, but not limited to, seconds, minutes, hours, and the like.

Continuing to refer to FIG. 1, in some embodiments, physiology affecting information 108 may include sleep data. "Sleep data" as used in this disclosure is information pertaining to the act of sleeping. Sleep data may include a location of sleep for an individual. A location of sleep may include geographical coordinates, such as longitudes, latitudes, and the like. A location of sleep may include addresses, such as streets, cities, states and/or countries, without limitation. A location of sleep may include locations of a user's bed, couch, and/or other resting location. In some embodiments, sleep data may include a time of sleep. A time of sleep may include a time a user may begin to fall asleep, for instance 10:45PM EST, or 2100 Hrs PT, without limitation. Sleep data may include a duration of sleep, such as seconds, minutes, hours, and the like. In some embodiments, sleep data may be generated by one or more sensors of wireless communication tag 104. The wireless communication tag 104 may include an accelerometer, heart rate sensor, and the like. In some embodiments, the wireless communication tag 104 may be configured to generate sleep data such as heart rate, heart rate variability, sleep stage determinations, and/or other data. In some embodiments, the wireless communication tag 104 may receive sleep data from one or more devices, such as, but not limited to, fitness devices.

The physiology affecting information 108 may be generated based on a location of the wireless communication tag 104. A location may include a geographical location (e.g., global positioning coordinates, map coordinates), approximate location (e.g., street address), relative location (e.g., a specific room in a house), and the like, without limitation. A location may be associated with a type of physiology affecting information 108. For instance, and without limitation, wireless communication tag 104 may be placed by a bed and assigned a corresponding location. In a further example, the wireless communication tag 104 or 104' may provide data that may be used to differentiate if a person is at home or traveling which can affect meal types, sleep quality, time zone changes, or the like, which can impact blood glucose levels and insulin delivery. Wireless communication tag 104 may, for example, generate physiology affecting information 108 to include the sleep data described above. Continuing this example, another wireless communication tag 104', different from the wireless communication tag 104, but in addition to, may generate physiology affecting information 108' to include meal data and be placed in or on a lunchbox or cooler. In an example, a wireless communication tag, such as 104 or 104' may include a global positioning system (GPS), cellular radio, Wi-Fi receiver, Bluetooth^{®} transceiver, and the like which may allow wireless communication tag to approximate its location. Wireless communication tag 104 may communicate with one or more external computing devices to update physiology affecting information 108 using the approximated location of wireless communication tag 104.

Still referring to FIG. 1, in some embodiments, user device 112 may be configured to generate a mode of operation 116 for drug delivery device 120 as a function of physiology affecting information 108. In some embodiments, drug delivery device 120 may include a wearable medical device. A "wearable medical device" as used in this disclosure is a device affixed to the skin of a user that monitors and/or effects a user's health. Drug delivery device 120 may include one or more needles, fluid chambers, actuators, and/or other elements that may be used to inject an amount of a liquid drug subcutaneously into a user. Examples of liquid drugs may include any drug in liquid form capable of being administered by a drug delivery device via a cannula, including, for example, insulin, glucagon-like peptide-1 (GLP-1), pramlintide, glucagon, co-formulations of two or more of GLP-1, pramlintide, and insulin; as well as pain relief drugs, such as opioids or narcotics (e.g., morphine, or the like), methadone, arthritis drugs, hormones, such as estrogen and testosterone, blood pressure medicines, chemotherapy drugs, fertility drugs, or the like. The drug delivery device 120 may include, without limitation, smart watches, heart rate monitors, electrocardiogram (EKG) devices, pace makers, and the like. In some embodiments, drug delivery device 120 may include one or more sensors. A "sensor" as used in this disclosure is a device capable of detecting natural phenomenon. Sensors may include, but are not limited to, thermometers, ohmmeters, blood glucose monitors, blood oxygenation sensors, heart rate monitors, and/or other sensors. A sensor of drug delivery device 120 may be configured to detect blood glucose, blood oxygen, temperature, heart rate, breathing rate, and/or other biological parameters of a user. In some embodiments, drug delivery device 120 may include one or more securing mechanisms. A "securing mechanism" as used in this disclosure is an element or group of elements that connect two or more objects together. Securing mechanisms may include, without limitation, screws, nuts, bolts, straps, clamps, adhesive pads, and the like. An adhesive patch may include, without limitation, epoxy adhesives, polyurethane adhesives, polyimide adhesives, and/or other materials.

Still referring to FIG. 1, a "mode of operation" as used in this disclosure is one or more parameters of a process. A process may include, but is not limited to, calculating insulin dosages, determining timing of insulin injections, analyzing blood samples, and the like. Parameters that may be changed through varying modes of operation 116 may include one or more settings of drug delivery device 120. Settings of drug delivery device 120 may include, but are not limited to, power consumption settings, such as processor operating frequencies, frequency of blood glucose sensing, occurrences of transmission between devices, and the like. In some embodiments, parameters may include, without limitation, current basal rates of insulin delivery, target blood glucose set points, automated insulin delivery (AID) constraints, insulin on board (IOB) constraints, and the like. Constraints may include, but are not limited to, time constraints, range constraints, and/or other values of one or more algorithms. Wireless communication tag 104 may transmit physiology affecting information 108 directly to drug delivery device 120. Drug delivery device 120 may select a mode of operation 116 based on the physiology affecting information 108. In some embodiments, wireless communication tag 104 may transmit physiology affecting information 108 with user device 112. User device 112 may select the mode of operation 116 and communicate the mode of operation 116 with drug delivery device 120. Wireless communication tag 104 may be configured to communicate physiology affecting information 108 with user device 112 and/or drug delivery device 120 based on a relative distance of user device 112 and/or drug delivery device 120 with respect to the wireless communication tag 104. In other words, wireless communication tag 104 may be configured to communicate physiology affecting information 108 with user device 112 and/or drug delivery device 120 when user device 112 and/or drug delivery device 120 is located within a communication range of the wireless communication tag 104. For instance, wireless communication tag 104 may be configured to communicate physiology affecting information 108 with user device 112 and/or drug delivery device 120 as a function of a proximity of user device 112 and/or drug delivery device 120 with wireless communication tag 104. A "proximity" as used in this disclosure is a measurement of physical distance between two or more objects. A proximity may include, without limitation, inches, feet, millimeters, centimeters, meters, and the like. For instance and without limitation, wireless communication tag 104 may communicate physiology affecting information 108 with user device 112 at a proximity of 10 or less centimeters between user device 112 and wireless communication tag 104. In embodiments, wireless communication tag 104 may communicate physiology affecting information 108 with user device 112 at a proximity of about less than 20 centimeters, specifically about less than 15 centimeters, and more specifically about less than 10 centimeters between user device 112 and wireless communication tag 104. Communication may occur through NFC, RFID, Bluetooth, and/or other forms of wireless communication. In other embodiments, the wireless communication tag 104 may communicate physiology affecting information 108 with the user device 112 at a proximity of approximately 10 or more centimeters between wireless communication tag 104 and user device 112. At larger distances, wireless communications may occur through Bluetooth and/or Wi-Fi connections.

Still referring to FIG. 1, the user device 112 and/or drug delivery device 120 may, for example, generate a meal time mode of mode of operation 116. The mode of operation 116 may include a meal time mode. A "meal time mode" as used in this disclosure is a form of programming of a computing device that assists a user with consumption of food items. A meal time mode of mode of operation 116 may include an adjustment of one or more settings of drug delivery device 120, such as, but not limited to, frequency of blood glucose sensing, an amount of insulin to be delivered as a bolus and a schedule for delivering the insulin, and/or other settings of drug delivery device 120. For instance, a meal time mode of mode of operation 116 may include adjusting an amount insulin to be delivered based on an amount of insulin onboard (IOB). A meal time mode of mode of operation 116 may include, but is not limited to, adjusting a total daily insulin (TDI) value, calculating one or more upfront boluses, adjusting IOB constraints, and the like. In a meal time mode of mode of operation 116, a blood glucose set point may be lowered which may allow for additional insulin delivery. User device 112 may be prompted to generate mode of operation 116 to include a meal time mode as a function of physiology affecting information 108. The physiology affecting information 108 may include a location of wireless communication tag 104. A location of wireless communication tag 104 may be associated with a consumption of one or more food items. The wireless communication tag 104 may be placed in or on, without limitation, lunch boxes, backpacks, cafeterias, snack pantry doors, grocery store aisles, vending machines, shelves, plates, cups, glasses, bottles, food containers, and/or other environments where a user may select and/or consume one or more food items. In some embodiments, the wireless communication tag 104 may be pre-programmed to include meal data of one or more meal items. The wireless communication tag 104 may be programmed through a wired connection, wireless connection, and the like. In some embodiments, wireless communication tag 104 may be programmed through a mobile application, web portal, and the like. Wireless communication tag 104 may be updated through one or more external computing devices, such as, but not limited to, servers, cloud-computing networks, smartphones, laptops, and the like. In some embodiments, wireless communication tag 104 may interlink with a network of a school cafeteria or other eating establishment. For example, interlinking with a network of a school cafeteria may allow wireless communication tag 104 to continuously update meal data of the one or more meals associated with the location of the wireless communication tag 104. A school cafeteria network may update wireless communication tag 104 to include meal data of one or more pre-made meals, meal stations of a kitchen, and the like. For instance, and without limitation, a plurality of wireless communication tags 104 may be positioned at a front of each station of a school cafeteria, wherein each wireless communication tag 104 may include and be configured to transmit meal data associated with that particular station of the school cafeteria. In another non-limiting example, wireless communication tag 104 may be pre-programmed to transmit meal data of the contents of a lunchbox when user device 112 is within a proximity of 4mm of wireless communication tag 104.

Still referring to FIG. 1, in some embodiments, a meal time mode may include user device 112 generating and/or displaying meal data through a graphical user interface (GUI). In some embodiments, a GUI (shown in a later example) of user device 112 and/or drug delivery device 120 may be configured to receive user input such as, but not limited to, mouse clicks, keyboard strokes, and the like. A GUI of user device 112 and/or the drug delivery device 120 may be configured to receive a touch input through a touch screen. The touch input may include taps, swipes, long presses, and the like. User device 112 may generate and/or animate a GUI as a function of a proximity to wireless communication tags 104 and 104'. User device 112 may receive user input through a GUI and adjust one or more settings of mode of operation 116 as a function of the user input.

With continued reference to FIG. 1, a user may enter meal data into a memory (shown in a later example) of wireless communication tag 104. Entering meal data into the memory of wireless communication tag 104 may include writing by transmitting meal data from a user device 112 to a transmission element and logic circuitry (shown in a later example) of wireless communication tag 104. For example, a user device 112 may write (it is understood that writing as referred to herein is via wireless transmission and not physically writing) to wireless communication tag 104 through, but not limited to, NFC writers, RFID writers, smartphones, and/or other computing devices. For instance, and without limitation, a user may write to wireless communication tag 104 through a mobile application interface (not shown in this example) of user device 112. For example, the user device 112 may include an NFC reader/writer that may enable a user to transmit to wireless communication tag 104 through user device 112. Continuing this example, a user may write meal data of the contents of a lunchbox to the wireless communication tag 104. The transmitted meal data may include macronutrients of an apple, juice box, and a ham and cheese sandwich on wheat bread. Macronutrients of an apple may include, for example, 0.3 grams of protein, 0.2 grams of fat, and 13.8 grams of carbohydrates. Macronutrients of a juice box may include, for example, 0 grams of protein, 0 grams of fat, and 26.3 grams of carbohydrates. Macronutrients of a ham and cheese sandwich on wheat bread may include, for example, 23 grams of protein, 9 grams of fat, and 29 grams of carbohydrates. A total macronutrient count of the meal may include, for example, 23.3 grams of protein, 9.2 grams of fat, and 69.1 grams of carbohydrates. Wireless communication tag 104 may store the macronutrients of the meal and communicate the macronutrients to user device 112 and/or other computing devices. Wireless communication tag 104 may store a total macronutrient value of a meal and/or individual macronutrient values of each item of a meal. User device 112 may receive from the wireless communication tag 104 a total macronutrient content of a meal and/or a breakdown of each macronutrient value of each item in the meal.

Still referring to FIG. 1, in some embodiments, user device 112 may generate an exercise mode of mode of operation 116. An "exercise mode" as used in this disclosure is a form of programming of a computing device that assists a user in physical exertion. An exercise mode of mode of operation 116 may include adjusting one or more parameters of drug delivery device 120. For instance, a target blood glucose level of the user may be adjusted, a drug delivery schedule may be updated, a frequency of blood glucose sensing may be adjusted, and the like. The user device 112 may automatically enable an activity mode of mode of operation 116 based on one or more detected thresholds such as, but not limited to, heart rate variability, heart rate, respiration rates, and the like. A blood glucose setpoint of an exercise mode of mode of operation 116 may be adjusted, such as lower, raised, and the like. For instance, a blood glucose setpoint of an exercise mode of mode of operation 116 may be reduced, which may help prevent hypoglycemia. A bolus amount of exercise mode of mode of operation 116 may be reduced which may account for an increase insulin sensitivity of a user post-exercise. An exercise mode of mode of operation 116 may adjusted one or more night time basal rates, such as, without limitation, lowering night time basal rights which may help prevent night time hypoglycemia post-exercise. Wireless communication tag 104 may be placed within an exercise facility, such as, but not limited to, recreational centers, playgrounds, sports fields, gyms, and the like. Wireless communication tag 104 may be pre-programmed to include exercise data associated with a location of exercise. For instance and without limitation, wireless communication tag 104 may be pre-programmed to include exercise data specific to an indoor basketball court. Exercise data may include types of exercises, duration of exercises, and the like, as described above. In some embodiments, wireless communication tag 104 may be placed by and/or near specific exercise equipment, such as, without limitation, stationary bikes, dumbbell racks, barbell racks, cross-fit equipment, and the like. Wireless communication tag 104 may be pre-programmed to include one or more parameters of operation of drug delivery device 120 associated with specific exercises. As a non-limiting example, physiology affecting information 108 communicated from the wireless communication tag 104 may include exercise data of a 1.5hr basketball game. In some embodiments, wireless communication tag 104 may be configured to communicate with a sports facility network, such as one or more cloud-computing devices, laptops, servers, smartphones, and the like. Wireless communication tag 104 may communicate exercise data with a sports facility such as, but not limited to, sporting event type, sporting event duration, and the like. For instance and without limitation, wireless communication tag 104 may be positioned next to an entrance of a basketball facility. Furthering this example, the wireless communication tag 104 may communicate with one or more external computing devices of the basketball facility to update exercise data related to the physiology affecting information to include sporting event types, sporting event durations, and the like. Through continually updating exercise data of the physiology affecting information 108, the wireless communication tag 104 may advantageously remain in a single location.

Still referring to FIG. 1, an exercise mode of mode of operation 116 may include raising and/or decreasing one or more target set points of blood glucose amounts and/or modifying one or more basal rates of insulin for a user of drug delivery device 120 based on the physiology affecting information 108 or 108' received from a wireless communication tag 104 or 104'. For instance and without limitation, a basal rate of insulin may be adjusted to approximately 24% for mild aerobic activity, approximately 50% for moderate aerobic activity, and/or approximately 75% for heavy aerobic activity. As another non-limiting example, a target set point of blood glucose for a user may include being set (when not in exercise mode) to 120 mg/dL and be adjusted, when the mode of operation 116 is exercise mode, to 100 mg/dL. An exercise mode of operation 116 may include a duration of exercise and/or a duration of a modified target set point of blood glucose, basal rates of insulin, and the like that may be modified based on the physiology affecting information 108 or 108' transmitted by the wireless communication tag 104 or 104'. Continuing the above example, a target set point of blood glucose for a user may drop to 100 mg/dL for a tunable or default duration of exercise and increase to 120 mg/dL after the expiration of the tuned or default duration. In some embodiments, an exercise mode of operation 116 may include suggesting, through a GUI of user device 112, that a user consume a certain amount and/or type of carbohydrate, protein, and/or fat before physical exertion. In some embodiments, upon reading the wireless communication tag 104 and in response to receipt of the physiology affecting information 108, the user device 112 may generate one or more user prompts through a GUI for a user to set drug delivery device 120 to an exercise mode. A user may interface with a GUI to confirm or reject a proposed exercise mode of drug delivery device 120. The user device 112 may generate a user pattern of exercise and/or confirmations of exercises, duration of exercises, adjustments of the drug delivery device 120, and the like. In some embodiments, the drug delivery device 120 includes, for example, a default setting to exit an exercise mode of mode of operation 116. A default setting may include a time period of, but not limited to, 1 hour, 2 hours, and the like. The user device 112 may generate a prompt for a user to confirm an exercise mode of mode of operation 116 after a time period from a rejected confirmation of the user and/or withhold from generating a user prompt for a certain period of time. For instance, and without limitation, a user may select through a GUI (shown in a later example) of the user device 112 that a suggested exercise mode of mode of operation 116 not be prompted for 2 hours, 6 hours, and the like.

Still referring to FIG. 1, in some embodiments, mode of operation 116 may include a driving mode. A "driving mode" as used in this disclosure is a form of programming of a computing device that assists a user with driving. Assisting a user with driving may include, but is not limited to, adjusting blood glucose target setpoints, measuring blood glucose levels, injecting medication, and the like, as described below. Wireless communication tag 104 may be configured to transmit physiology affecting information 108, which may include driving data. Driving data may include data as described above, without limitation. In some embodiments, a wireless communication tag 104 may be positioned within a car, truck, motorcycle, bike, scooter, and/or other vehicle, without limitation. In some embodiments, wireless communication tag 104 may be preprogrammed to include driving data of specific vehicles, which may be transmitted through physiology affecting information 108 to user device 112. In some embodiments, the wireless communication tag 104 is configured to communicate with one or more external computing devices, such as, but not limited to, on-board processors of one or more vehicles, a global positioning system (GPS), cellular networks, and/or other devices. Wireless communication tag 104 may be configured to determine a driving speed of one or more vehicles through an external computing device. in response to reading a wireless communication tag 104 on a vehicle, a user device executing an AID algorithm may be configured to prompt a driving mode of mode of operation 116 as a function of a driving speed of a vehicle. For instance and without limitation, wireless communication tag 104 may detect a driving speed of 20 miles per hour (mph) and communicate physiology affecting information 108 including driving data to user device 112, which may prompt a user to select a driving mode of mode of operation 116 through a GUI of user device 112.

Still referring to FIG. 1, in some embodiments, a driving mode of mode of operation 116 may include an adjustment to one or more target setpoints of blood glucose of a user, basal rates, and the like, through drug delivery device 120. A diabetic user may have or potentially have a hypoglycemic event during driving of a vehicle which may be a safety hazard. Drug delivery device 120 may include one or more sensors that may monitor blood glucose of a user during an operation of a vehicle, such as driving a car. A driving mode of mode of operation 116 may include setting drug delivery device 120 to increase a frequency of measuring a blood glucose and/or other biological parameter of a user which may help in detecting early signs of hypoglycemia. In some embodiments, a driving mode of mode of operation 116 may include adjusting a target setpoint of to increase or decrease, such as, without limitation, by 10mg/dL, 15mg/dL, or other amounts of blood glucose. Additionally, or alternatively, other setpoints or parameters of an AID algorithm may be modified in response to the driving mode. In some embodiments, a driving mode of mode of operation 116 may include alerting a user to consume a carbohydrate, fat, and/or protein amount through user device 112. An "alert" as used in this disclosure is a warning generated by a computing device. An alert may include, without limitation, an audible alert such as a ringing, chime, chirp, and the like. An audible alert my play through user device 112 and/or through an audio system of a vehicle operated by a user. In some embodiments, an alert may include a visual alert. A visual alert may include one or more light emitting diode (LED) flashes, high contrasted images flashed on a GUI, text, and/or other forms of alerting messages. A visual alert may be shown through user device 112 and/or one or more screens of a vehicle operated by a user. In some embodiments, a visual alert may include one or more phrases, such as "Blood Glucose Low!". In some embodiments, an alert may include a vibratory alert. A vibratory alert may include an oscillating motion of one or more motors, rotors, and the like. In some embodiments, a vibratory alert may be presented through user device 112 and/or through an interface of a vehicle, such as a steering wheel or seat, without limitation. A driving mode of mode of operation 116 may include a user prompt generated through a GUI of user device 112 asking a user to set a driving mode of drug delivery device 120. A user may confirm a driving mode of drug delivery device 120 or reject a driving mode of drug delivery device 120. Upon confirmation of a driving mode, the drug delivery device 120 may increase a sensing time and/or frequency of a user's blood glucose, heart rate, and the like. In some embodiments, user device 112 may determine a user pattern of driving mode confirmations, such as, but not limited to, duration of operation, vehicle type, dates, and the like. User device 112 may be configured to present a user prompt to enter a driving mode during determined acceptable conditions of a user pattern, such as, without limitation, time of day, current blood glucose level, duration of drive, and the like.

Still referring to FIG. 1, in some embodiments, mode of operation 116 may include a sleep mode. A "sleep mode" as used in this discourse is a function of a computing device that aids a user with sleep. A sleep mode of mode of operation 116 may include adjusting one or more parameters of mode of operation 116 such as, but not limited to, basal rates, blood glucose set points, IOB constraints, and the like. In some embodiments, a user may set a night time basal rate for a specific set point, such as at a specific time, period of time, and the like. A sleep mode may calculate basal needs of a user based on previous insulin delivery while the user may be sleeping. Wireless communication tag 104 may be positioned in a location associated with a user's sleep, such as, but not limited to, beds, couches, hotel rooms, and the like. In some embodiments, wireless communication tag 104 may communicate with one or more external computing devices of a location of sleep, such as, without limitation, servers, laptops, desktops, cloud-computing networks, and the like. Wireless communication tag 104 may receive and/or communicate sleep data with one or more external computing devices. Sleep data may include geographical locations, duration of sleep, time of day, and the like. Sleep data may be as described above, without limitation.

Still referring to FIG. 1, in some embodiments, wireless communication tag 104 may be pre-programmed with sleep data, such as, but not limited to, duration of sleep, location, and the like. In other embodiments, wireless communication tag 104 may update sleep data with one or more external computing devices. Wireless communication tag 104 may communicate physiology affecting information 108, which may include sleep data, with user device 112. User device 112 may generate a sleep mode of mode of operation 116 as a function of physiology affecting information 108. A sleep mode may include adjusting one or more target setpoints of blood glucose of a user, adjusting one or more basal rates of insulin for a user, suspension of automated insulin doses, and the like through drug delivery device 120. A diabetic user may be prone to hypoglycemia during periods of sleep. Drug delivery device 120 may adjust amounts of insulin delivered to a user to prevent hypoglycemia during a user's sleep. In some embodiments, drug delivery device 120 may increase a frequency of measurement of a user's blood glucose through one or more sensors in a sleep mode. In other embodiments, drug delivery device 120 may decrease a frequency of measurement of a user's blood glucose in a sleep mode. User device 112 may generate a prompt for a user to set drug delivery device 120 into mode of operation 116 of a sleep mode. A user may confirm or reject a sleep mode of mode of operation 116 of drug delivery device 120. User device 112 may determine a pattern of sleep data of a user and generate user prompts as a function of the pattern of sleep data.

Still referring to FIG. 1, in some embodiments, user device 112 may be configured to determine mode of operation 116 of drug delivery device 120 as a function of a historical trend of a plurality of modes of operation 116 of drug delivery device 120. A "historical trend" as used in this disclosure is a pattern of data that occurred in the past. User device 112 may determine mode of operation 116 should be set to one or more of a meal time mode, sleeping mode, exercise mode, driving mode, and the like as a function of one or more parameters such as time of day, blood glucose levels, basal rates of insulin, and the like. For instance, and without limitation, user device 112 may determine that on Tuesdays after a meal time mode set at 12:15PM an exercise mode should be set at around 6:00P.M. User device 112 may actively search for wireless communication tag 104 based on historical trends to confirm one or more user patterns.

As mentioned above, the drug delivery device 120 may be coupled by adhesive to the skin of a user's body. Additionally, or alternatively, the medical injection device 120 may be a multi-part device. For example, the drug delivery device 120 may have a first part and a second part that couple or connect together. The first part and/or second part may fit into or slide into a tray or cradle that is adhered to the user's body, and the first part and/or second part may be removable from the tray. If using a first part and a second part, the first part may comprise reusable components (e.g., electronic circuitry, processor, memory, a drive mechanism, and potentially a rechargeable battery), and the second part may comprise disposable components (e.g., a reservoir, a needle and/or cannula, a disposable battery, and other portions or components that come into contact with the liquid drug or medicament). Moreover, the first part and the second part may contain their own housing or may combine together to form a single housing. The drug delivery device 120 may be directly coupled to a user (e.g., directly attached to a body part and/or skin of the user via an adhesive, directly, via the tray, or the like). In an example, a surface of the drug delivery device 120 or a tray into which the drug delivery device 120 couples may include an adhesive to facilitate attachment to the skin of a user.

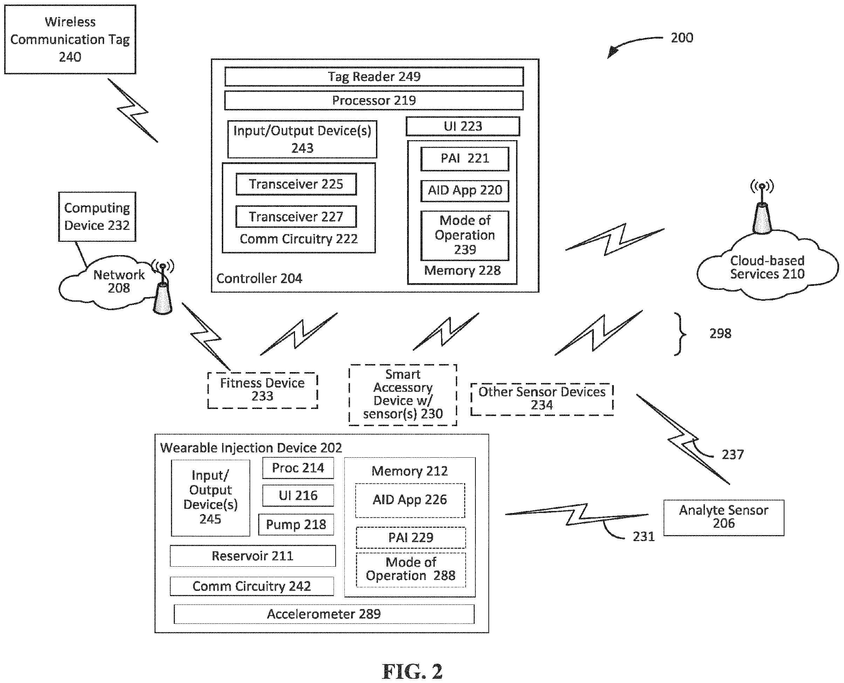

A block diagram of a drug delivery system 200 is illustrated in FIG. 2. In some examples, the drug delivery system 200 is suitable for delivering insulin to a user in accordance with the disclosed embodiments. The drug delivery system 200 may include one or more of a wearable injection device 202, a wireless communication tag 240, a controller 204 and an analyte sensor 206. In addition, the drug delivery system 200 may interact with a computing device 232 via a network 208 as well as obtain or contribute to cloud-based services 210 as well as other devices, such as a fitness device 233, smart accessory device with sensor(s) 230 and other sensor devices 234, such as blood oxygen sensor, stress sensor, or the like. Drug delivery system 200 may be configured to operate in one or more modes of operation, as described above with reference to FIG. 1.

Still referring to FIG. 2, the wearable injection device 202 may be a wearable device that is worn on the body of the user. The wearable injection device 202 may be directly coupled to a user (e.g., directly attached to the skin of the user via an adhesive, or the like at various locations on the user's body, such as thigh, abdomen, or upper arm). In an example, a surface of the wearable injection device 202 may include an adhesive to facilitate attachment to the skin of a user.

The wearable injection device 202 may also include a processor 214. The processor 214 may be implemented in hardware, software, or any combination thereof. The processor 214 may, for example, be a microprocessor, a logic circuit, a field programmable gate array (FPGA), an application specific integrated circuit (ASIC) or a microprocessor coupled to a memory. The processor 214 may maintain a date and time as well as be operable to perform other functions (e.g., calculations or the like). The processor 214 may be operable to execute an AID application 226 based on physiology affecting information (PAI) 229 and/or a mode of operation 288 stored in the memory 212 that enables the processor 214 to direct operation of the wearable injection device 202. The AID application 226 may control insulin delivery to the user per an AID algorithm. The memory 212 may store AID application settings for a user, such as specific factor settings, and AID algorithm settings, such as maximum insulin delivery, insulin sensitivity settings, total daily insulin (TDI) settings and the like as well as settings such as a selected or generated setting for mode of operation 288, or PAI 229 data. The wearable injection device 202 may also have an accelerometer 289 that provides signals to the processor 214 and/or the controller 204 related to motion of the wearable injection device 202.

The analyte sensor 206 may be operable to collect physiological condition data, such as the blood glucose measurement values and a timestamp, ketone levels, heart rate, blood oxygen levels and the like that may be shared with the wearable injection device 202, the controller 204 or both. For example, the communication circuitry 242 of the wearable injection device 202 may be operable to communicate with the analyte sensor 206 and the controller 204 as well as the devices 230, 233 and 234. The communication circuitry 242 may be operable to communicate via Bluetooth^{®}, Wi-Fi, a near-field communication standard, a cellular standard, or any other wireless protocol. The analyte sensor 206 and/or the controller 204 may be configured with communication circuitry similar to the communication circuitry 242.

Still referring to FIG. 2, the input/output device(s) 245 may one or more of a microphone, a speaker, a vibration device, a display, a push button, a touchscreen display, a tactile input surface, or the like. The input/output device(s) 245 may be coupled to the processor 214 and may include circuitry operable to generate signals based on received inputs and provide the generated signals to the processor 214. In addition, the input/output device(s) 245 may be operable to receive signals from the processor 214 and, based on the received signals, generate outputs via a respective output device.

The wearable injection device 202 may include a reservoir 211. The reservoir 211 may be operable to store drugs, medications or therapeutic agents suitable for automated delivery, such as insulin, morphine, methadone, hormones, glucagon, glucagon-like peptide, blood pressure medicines, chemotherapy drugs, combinations of drugs, such as insulin and glucagon-like peptide, or the like as well as those described with reference to FIG. 1. A fluid path from the reservoir 211 to the user may be provided via tubing, couplings and a needle/cannula (not shown). The wearable injection device 202 may be operable based on control signals from the processor 214 to expel the drugs, medications or therapeutic agents, such as insulin, from the reservoir 211 to deliver doses of the drugs, medications or therapeutic agents, such as the insulin, to the user via the fluid path. For example, the processor 214 by sending control signals to the pump 218 may be operable to cause insulin to be expelled from the reservoir 211.
There may be one or more communication links 298 with one or more devices physically separated from the wearable injection device 202 including, for example, a controller 204 of the user and/or a caregiver of the user and/or a sensor 206. The analyte sensor 206 may communicate with the wearable injection device 202 via a wireless communication link 231 and/or may communicate with the controller 204 via a wireless communication link 237. The communication links 231, 237, and 298 may include wired or wireless communication paths operating according to any known communications protocol or standard, such as Bluetooth, Wi-Fi, a near-field communication standard, a cellular standard, or any other wireless protocol.

Still referring to FIG. 2, the wearable injection device 202 may also include a user interface (UI) 216, such as an integrated display device for displaying information to the user, and in some embodiments, receiving information from the user. For example, the UI 216 may include a touchscreen and/or one or more input devices, such as touchscreen, buttons, knob or a keyboard that enable a user to provide an input. The UI 216 may include a graphical user interface (GUI). A "graphical user interface" as used in this disclosure is a form of interface that allows one or more users to interact with a computing device through graphical icons. A GUI may include one or more event handlers. An "event handler" as used in this disclosure is a callback routine that operates asynchronously once an event takes place. An event handler of a GUI of wearable injection device 202 may be linked with one or more graphical icons of the GUI. For instance, and without limitation, an event handler may be programmed to generate a pop-up window of a settings menu upon a click of a settings icon displayed through a GUI. User device 112, as described above with reference to FIG. 1, may include a GUI that may be programmed to receive user input. Fitness device 233, smart accessory device 230, and/or other sensor devices 234 may be configured to receive user input, such as through a GUI, without limitation.

Additionally, referring still to FIG. 2, controller 204 may be configured to display a bolus calculator through UI 223. A "bolus calculator" as used throughout this disclosure is a program that determines a quantity of medicine to deliver to a user. Controller 204 may access a bolus calculator program stored in memory 228. Alternatively, and without limitation, controller 204 may access a bolus calculator through cloud-based services 210, such as through sending one or more values to cloud-based services 210 and receiving an output of a bolus calculation performed through cloud-based services 210. A bolus calculator may be configured to calculate an amount of insulin to deliver to a user through wearable injection device 202. A bolus calculator may be configured to calculate an insulin delivery as a function of meal data communicated through physiology affecting information (PAI) 221. PAI 221 may include physiology affecting information 108 or 108' as described above with reference to FIG. 1. Meal data may include food types, macronutrients, micronutrients, and the like, as described above. A bolus calculator may input an amount of carbohydrates, proteins, fats, portion sizes, current blood glucose of a user, and the like, and output an amount and/or rate of insulin to be delivered to a user. In some embodiments, UI 223 may generate and/or display meal data along with a user prompt asking to confirm operation of a bolus calculator. Upon confirmation of the user prompt, UI 216 may prepopulate a bolus calculation of insulin to be delivered through wearable injection device 202. Prepopulation may include entering one or more values into one or more user text fields of UI 223 before a user interacts with the one or more user text fields. In other embodiments, a user may decline confirmation to proceed with a bolus calculation through UI 223, to which a user device, such as user device 112 as described above with reference to FIG. 1 and/or wearable injection device 202 may return to a default state. In some embodiments, user device 112 may store meal data of physiology affecting information and user confirmations of bolus calculations associated with particular meal data. The user device 112 may determine a pattern of user confirmations and generate user prompts as a function of the pattern of user confirmations. For instance, and without limitation, a user may continuously decline a confirmation of using a bolus calculator for a snack of a granny smith apple on Tuesday evenings. For example, the user device 112 may stop generating user prompts for a bolus calculation when food, such as a granny smith apple, is detected through meal data of physiology affecting information on Tuesday evenings. A user may override this behavior of the user device at any point and/or select, through UI 223.

Still referring to FIG. 2, in addition, the processor 214 may be operable to receive data or information from the analyte sensor 206 as well as other devices, such as smart accessory device 230, fitness device 233 or another wearable device 234 (e.g., a blood oxygen sensor or the like), that may be operable to communicate with the wearable injection device 202. For example, fitness device 233 may include a heart rate sensor and be operable to provide heart rate information or the like to the controller 204, wearable injection device 202 and/or analyte sensor 206.

Still referring to FIG. 2, the wearable injection device 202 may interface with a network 208. The network 208 may include a local area network (LAN), a wide area network (WAN) or a combination therein and be operable to wirelessly couple to the wearable injection device 202, the controller, and devices 230, 233, and 234. A computing device 232 may be interfaced with the network 208, and the computing device may communicate with the insulin delivery device 202. The computing device 232 may be a healthcare provider device, a guardian's computing device, or the like through which a user's controller 204 may interact to provide or obtain information, store or receive settings, and the like. The AID application 220 may be operable to execute an AID algorithm and present a graphical user interface via communication circuitry 222 and network 208 on the computing device 232 enabling the input and presentation of information related to the AID algorithm.

Still referring to FIG. 2, the drug delivery system 200 may include an analyte sensor 206 for detecting the levels of one or more analytes of a user, such as blood glucose levels, ketone levels, other analytes relevant to an insulin treatment program, or the like. The analyte level values detected may be used as physiological condition data, and be sent to the controller 204 and/or the wearable injection device 202. The analyte sensor 206 may be coupled to the user by, for example, adhesive or the like and may provide information or data on one or more medical conditions and/or physical attributes of the user. The analyte sensor 206 may be a continuous glucose monitor (CGM), ketone sensor, or another type of device or sensor that provides blood glucose measurements that is operable to provide blood glucose concentration measurements. The analyte sensor 206 may be physically separate from the wearable injection device 202 or may be an integrated component thereof. The analyte sensor 206 may provide the processor 214 and/or processor 219 with physiological condition data indicative of measured or detected blood glucose levels of the user. The information or data provided by the analyte sensor 206 may be used in combination with the physiology affecting information 221 or 229 and/or the mode of operation 239 or 288 to modify an insulin delivery schedule and thereby cause the adjustment of drug delivery operations of the wearable injection device 202.

Still referring to FIG. 2, in the depicted example, the controller 204 may include a processor 219, a tag reader 249, input/output device(s) 243, communication circuitry 222, a user interface 223, and a memory 228. The controller 204 may be a special purpose device, such as a dedicated personal diabetes manager (PDM) device. The controller 204 may be a programmed general-purpose device that is a portable electronic device, such as any portable electronic device, smartphone, smartwatch, fitness device, tablet or the like including, for example, a dedicated processor, such as processor, a micro-processor or the like. The controller 204 may be used to program or adjust operation of the wearable injection device 202 and/or the analyte sensor 206. The processor 219 may execute processes to manage a user's blood glucose levels and that control the delivery of the drug or a therapeutic agent (e.g., a liquid drug or the like as mentioned above) to the user. The processor 219 may also be operable to execute programming code stored in the memory 228. For example, the memory 228 may be operable to store an AID application 220 for execution by the processor 219. The AID application 220 may be responsible for controlling the wearable injection device 202, including the automatic delivery of insulin based on recommendations and instructions from the AID algorithm, such as those recommendations and instructions described herein.

Still referring to FIG. 2, the memory 228 may store one or more applications, such as an AID application 220, and mode of operation 239 which may be the same as, or substantially the same as those described above with reference to the insulin delivery device 202. In addition, the settings 221 may store information, such as drug delivery history, blood glucose measurement values over a period of time, total daily insulin values, and the like. In addition, the memory may store AID settings and parameters, insulin treatment program history (such as insulin delivery history, blood glucose measurement value history and the like. Other parameters such as insulin-on-board (IOB) and insulin-to-carbohydrate ratio (ICR) may be retrieved from prior settings and insulin history stored in memory. For example, the AID application 220 may be operable to store the AID algorithm settings, such as blood glucose target set points, insulin delivery constraints, basal delivery rate, insulin delivery history, wearable injection device status, and the like. The memory 228 may also be operable to store data such as a food database for carbohydrate (or macronutrient) information of food components (e.g., grilled cheese sandwich, coffee, hamburger, brand name cereals, or the like). The memory 228 may be accessible to the AID application 220.

Still referring to FIG. 2, the input/output device(s) 243 of the controller 204 may one or more of a microphone, a speaker, a vibration device, a display, a push button, a tactile input surface, or the like. The input/output device(s) 243 may be coupled to the processor 219 and may include circuitry operable to generate signals based on received inputs and provide the generated signals to the processor 219. In addition, the input/output device(s) 243 may be operable to receive signals from the processor 219 and, based on the received signals, generate outputs via one or more respective output devices, such as a speaker, a vibration device, or a display.

The controller 204 may include a user interface (UI) 223 for communicating visually with the user. The user interface 223 may include a display, such as a touchscreen, for displaying information provided by the AID application 220. The touchscreen may also be used to receive input when it is a touch screen. The user interface 223 may also include input elements, such as a keyboard, button, knob or the like. In an operational example, the user interface 223 may include a touchscreen display controllable by the processor 219 and be operable to present the graphical user interface, and in response to a received input (audio or tactile), the touchscreen display is operable present a graphical user interface related to the received input.

The controller 204 may be operable to interface via a wireless communication link of the wireless communication links 298 with a network, such as a LAN or WAN or combination of such networks that provides one or more servers or cloud-based services 210 via communication circuitry 222. The communication circuitry 222, which may include transceivers 227 and 225, may be coupled to the processor 219. The communication circuitry 222 may be operable to transmit communication signals (e.g., command and control signals) to and receive communication signals (e.g., via transceivers 227 or 225) from the wearable injection device 202 and the analyte sensor 206. In an example, the communication circuitry 222 may include a first transceiver, such as 225, that may be a Bluetooth transceiver, which is operable to communicate with the communication circuitry 222 of the wearable injection device 202, and a second transceiver, such as 227, that may be a cellular transceiver, a Bluetooth^{®} transceiver, a near-field communication transceiver, or a Wi-Fi transceiver operable to communicate via the network 208 with computing device 232 or with cloud-based services 210. While two transceivers 225 and 227 are shown, it is envisioned that the controller 204 may be equipped more or less transceivers, such as cellular transceiver, a Bluetooth transceiver, a near-field communication transceiver, or a Wi-Fi transceiver.

Still referring to FIG. 2, the cloud-based services 210 may be operable to store user history information, such as blood glucose measurement values over a set period of time (e.g., days, months, years), a drug delivery history that includes insulin delivery amounts (both basal and bolus dosages) and insulin delivery times, types of insulin delivered, indicated meal times, blood glucose measurement value trends or excursions or other user-related diabetes treatment information, specific system settings including default settings, present settings and past settings, or the like.

In FIG. 2, other devices, like smart accessory device 230 (e.g., a smartwatch or the like), fitness device 233 and other wearable device 234 may be part of the drug delivery system 200. These devices may communicate with the wearable injection device 202 to receive information and/or issue commands to the wearable injection device 202. These devices 230, 233 and 234 may execute computer programming instructions to perform some of the control functions otherwise performed by processor 214 or processor 219. These devices 230, 233 and 234 may include user interfaces, such as touchscreen displays for displaying information such as current blood glucose level, insulin on board, insulin deliver history, or other parameters or treatment-related information and/or receiving inputs. The display may, for example, be operable to present a graphical user interface for providing input, such as request a change in basal insulin dosage or delivery of a bolus of insulin. Devices 230, 233 and 234 may also have wireless communication connections with the sensor 206 to directly receive blood glucose level data as well as other data, such as user history data maintained by the controller 204 and/or the wearable injection device 202.

The user interface 223 may be a touchscreen display controlled by the processor 219, and the user interface 223 is operable to present a graphical user interface that offers an input of a subjective insulin need parameter usable by the AID application 220. The processor 219 may cause a graphical user interface to be presented on the user interface 223. Different examples of the graphical user interface may be shown with respect to other examples. The AID application 220 may generate instructions for the pump 218 to deliver basal insulin to the user or the like.

In the controller 204, the processor 219 is also operable to collect physiological condition data related to the user from sensors, such as the analyte sensor 206 or heart rate data, for example, from the fitness device 233 or the smart accessory device 230. The processor 219 may output a control signal via one of the transceivers 225 or 227 to the wearable injection device 202. The outputted signal may cause the processor 214 to deliver command signals to the pump 218 to deliver an amount of related to the determined dosage of insulin in the reservoir 211 to the user based on an output of the AID algorithm. The processor 219 may also be operable to perform calculations regarding settings of the AID algorithm as discussed as herein. Modifications to the AID algorithm settings provided via the physiology affecting information (PAI) 221, such as described herein, that may be stored in the memory 228.

Referring now to FIG. 3, an embodiment of wireless communication tag 300 is presented. Wireless communication tag 300 may include a housing that may retain one or more internal components of wireless communication tag 300 in a secure position. A housing may include a shape such as, but not limited to, square, circular, triangular, ovular, and/or other shapes. In some embodiments, a housing of wireless communication tag 300 may include a material, such as, but not limited to, plastic, metal, glass, and/or any combination thereof. A housing of wireless communication tag 300 may include one or more colors, such as, but not limited to, red, green, blue, yellow, and/or any combination thereof. A housing of wireless communication tag 300 may include a first color denoting a top side of wireless communication tag 300 and a second color denoting a bottom side of wireless communication tag 300, for instance, and without limitation, white and black. In some embodiments, a housing of wireless communication tag 300 may include one or more securing mechanisms, such as, but not limited to, hooks, clamps, adhesive patches, and the like. In some embodiments, wireless communication tag 300 may include a metallic clip that may affix wireless communication tag 300 to one or more objects. Wireless communication tag 300 may include an adhesive pad that may stick wireless communication tag 300 to one or more surfaces, such as, but not limited to, walls, floors, poles, and the like. In some embodiments, wireless communication tag 300 may include, without limitation, an NFC tag, RFID tag, smart tag, and the like.

Still referring to FIG. 3, in some embodiments, wireless communication tag 300 may include logic circuitry 304. "Logic circuitry" as used in this disclosure is one or more elements configured to perform a function. The one or more elements of the logic circuitry 304 may include one or more resistors, capacitors, inductors, transistors, processors, microprocessors, controllers, microcontrollers, system on a chip (SoC), field programmable arrays (FPGAs), programmable logic devices (PLDs), integrated circuits (IC), and the like, without limitation. In some embodiments, the logic circuitry 304 may be communicatively connected to memory 308. As used in this disclosure, "communicatively connected" means connected by way of a connection, attachment, or linkage between two or more related elements which allows for reception and/or transmission of information therebetween. For example, and without limitation, this connection may be wired or wireless, direct, or indirect, and between two or more components, circuits, devices, systems, and the like, which allows for reception and/or transmission of data and/or signal(s) therebetween. Data and/or signals therebetween may include, without limitation, electrical, electromagnetic, magnetic, video, audio, radio frequency, and microwave data and/or signals, combinations thereof, and the like. A communicative connection may be achieved, for example and without limitation, through wired or wireless electronic, digital or analog communication, either directly or by way of one or more intervening devices or components. Further, communicative connection may include electrically coupling or connecting at least an output of one device, component, or circuit to at least an input of another device, component, or circuit. For example, and without limitation, via a bus or other facility for intercommunication between elements of a computing device. Communicative connecting may also include indirect connections via, for example and without limitation, wireless connection, radio communication, low power wide area network, optical communication, magnetic, capacitive, or optical coupling, and the like. In some instances, the terminology "communicatively coupled" may be used in place of communicatively connected in this disclosure.

In FIG. 3, the memory 308 may include, without limitation, random-access memory (RAM), dynamic random-access memory (DRAM), solid state drives (SSD), reserved memory, tag identifier (TID) memory, user memory, and the like, without limitation. In some embodiments, memory 308 may include one or more instructions configuring logic circuitry 304 to perform various tasks. Memory 308 may store and/or receive instructions from an external computing device, logic circuity 304, and the like.

Still referring to FIG. 3, in some embodiments, wireless communication tag 300 may include transmission element 312. A "transmission element" as used in this disclosure is an electronic device that can send and/or receive signals. Transmission element 312 may include one or more antennas, such as, but not limited to, monostatic antennas, bistatic antennas, omnidirectional antennas, linear antennas, planar antennas, loop antennas, reader antennas and/or writer antennas. Antennas of transmission element 312 may be composed of one or more wires, such as copper wires, silver wires, and the like. Transmission element 312 may be configured to receive and/or transmit radio signals. In some embodiments, transmission element 312 may be configured to receive and/or transmit radio signals in a range of 1Hz-2GHz. Transmission element 312 may receive and/or transmit radio signals below 1Hz and/or above 2GHz. Wireless communication tag 300 may be configured to interact with one or more devices over low frequency ranges of about 125Hz to 134KHz (LF), high frequency ranges of about 10MHz to 20MHz (HF), ultra-high frequency ranges of about 433MHz to 956MHz (UHF), and the like.

Still referring to FIG. 3, the wireless communication tag 300 may include power source 316. A "power source" as used in this disclosure is an origination of energy. Power source 316 may include an active power source, such as, without limitation, one or more batteries, capacitors, inductors, and the like. In some embodiments, power source 316 may include a passive power source. A passive power source may include one or more electrical components, such as energy harvesting circuits, configured to receive or extract energy from an external source, such as, without limitation, a smartphone, smartwatch, medical device, and the like. For example, a passive power source 316 may be configured to receive power in a form of one or more electromagnetic fields generated from a "reader device," such as a RFID-equipped smartphone during an interaction between the smartphone and transmission element 312. For instance, and without limitation, power source 316 may receive power from a smartphone interacting with logic circuitry 304 through transmission element 312, which may occur during a "reading" of wireless communication tag 300. For instance, and without limitation, one or more electromagnetic waves may be received through transmission element 312. The one or more electromagnetic waves may induce a current and/or voltage in one or more conductive elements of power source 316, which power source 316 may utilize to power components of wireless communication tag 300. Power source 316 may power logic circuitry 304, memory 308, and/or transmission element 312.

Still referring to FIG. 3, wireless communication tag 300 may be configured to operate in one or more states. A state of wireless communication tag 300 may include a sleep mode, in which wireless communication tag 300 enters a low or no-power state. Wireless communication tag 300 may remain in a sleep mode for a predetermined amount of time, a predetermined number of processor clock cycles, such as 3.3 GHz, 3.5 GHz, 16 MHz, 24 MHz or the like. In some embodiments, wireless communication tag 300 may awake from a sleep mode in response to an event. For example, and without limitation, wireless communication tag 300, may be configured to awake from a low power sleep mode every X (integer such as 1, 50 or the 1000) number of hours or minutes, seconds, milliseconds (or smaller units), after a timer times out, after a number of processor clock cycles (e.g., 3,300,000,000, 16 million or the like) or counts of the number of occurrences of an event, e.g., 10 times. An event may be a condition detected by transmission element 312, such as a certain value or range of values of amplitude, frequency, and the like, of an electromagnetic wave. An electromagnetic wave may be generated from one or more external devices. For instance, and without limitation, an event may include transmission element 312 receiving a 9Khz electromagnetic wave with a peak-to-peak amplitude of 1.2 volts. While wireless communication tag 300 is in a sleep mode, wireless communication tag 300 may be awakened, such as transitioning into an awake mode. An awake mode may include logical circuitry 304 performing one or more processes. Wireless communication tag 300 may awake in response to a passing of a number of processor clock cycles, if a timer times out, during a detection of an event, and/or a combination of these or other conditions.

Still referring to FIG. 3, the transmission element 312 may be configured to respond to a read request by one or more external computing devices, such as a user device as described above. A "data connection" as used in this disclosure is a form of interaction in which two or more parties share information. A data connection may include, without limitation, Bluetooth connections, Wi-Fi connections, and/or other connections. Transmission element 312 may include transmission circuitry that may enable communications over one or more wireless communication protocols. For instance, and without limitation, transmission circuitry of transmission element 312 may include one or more antennas, receivers, and the like. Antennas and receivers may include one or more coils of conductive elements, such as, but not limited to, copper. Transmission element 312 may include transmission circuitry that allows for transmission of electromagnetic waves of about 2.4GHz to 5GHz. In some embodiments, logic circuitry 304 may be configured to determine, through transmission element 312, a proximity to a user device and establish a data connection as a function of the proximity. In some embodiments, wireless communication tag 300 may be configured to establish a data connection with a wearable medical device. In an operational example, the logic circuitry 304 may be configured to receive meal data from an external computing device. The received meal data may be stored in the memory 308 as physiology affecting information (PAI) 309. In response to a read request from a user device or wearable medical device, the logic circuitry 304 may be able to retrieve the physiology affecting information 309 from the memory 308. Logic circuitry 304 may, for example communicate, through transmission element 312, the physiology affecting information 309 with a user device and/or a wearable medical device. Logic circuitry 304 may be configured to communicate a physiology affecting information 309 as a function of a location of wireless communication tag 300. For instance, wireless communication tag 300 may be positioned in a lunch box, to which logic circuitry 304 may be configured to communicate a physiology affecting information 309 including meal data associated with the lunch box.

Still referring to FIG. 3, in some embodiments, logic circuitry 304 may be configured to enable a user device to generate a GUI for a mode of operation of a wearable medical device as a function of a data communication. Enabling a user device may include providing the user device with data through a data connection which may allow the user device to populate one or more parts of a GUI.

With continued reference to FIG. 3, a user may "write" or otherwise communicate data, such as PAI 309, to wireless communication tag 300 through transmission element 312. A user may write specific PAI 309 for one or more locations of wireless communication tag 300. For instance, and without limitation, a user may write and/or program wireless communication tag 300 with driving data, using an NFC, RFID, and/or other device. An NFC, RFID, and/or other device may communicate a signal with transmission element 312. Transmission element 312 may communicate the signal with logic circuitry 304. Logic circuitry 304 may process and/or analyze the signal and store data of the signal in memory 308. PAI 309 may include a duration of a trip of a truck a user may operate. Wireless communication tag 300 may be placed in a console of a pick-up truck. A user device may interact with transmission element 312 of wireless communication device 300, such as, without limitation, when the user device becomes within a certain proximity with wireless communication tag 300 in the console of the pick-up truck. A user device may engage in one or more operations based on the PAI 309 communicated through transmission element 312, in this case driving data. Operations may include activating one or more modes of operation 116 as described above with reference to FIG. 1. In other examples, a user may write PAI 309 to memory 308 of wireless communication tag 300 for locations such as, but not limited to, beds, cafeterias, lunch boxes, gym equipment, and the like.

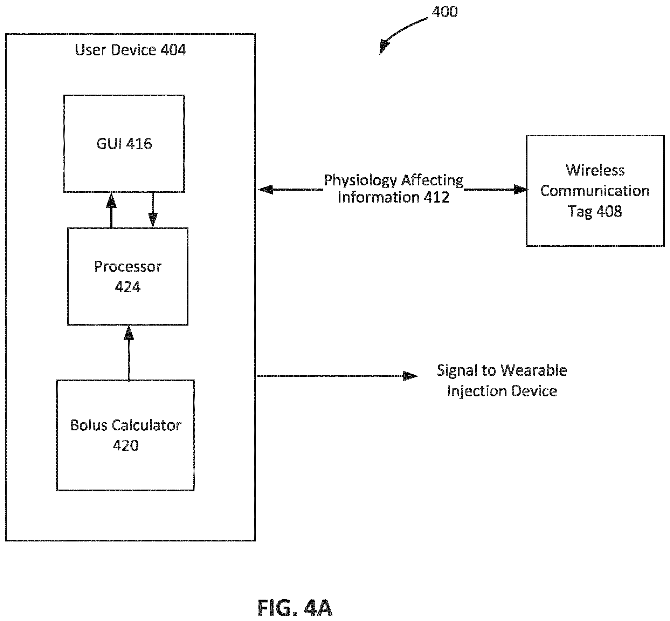

Referring now to FIG. 4A, exemplary embodiment of a drug delivery system 400 that utilizes physiology affecting information for wearable medical devices is presented. The system 400 may include user device 404, and one or more wireless communication tag 408, and/or physiology affecting information 412, which may be as described above without limitation in FIGS 1-3. User device 404 may enter a proximity to wireless communication tag 408, such as, but not limited to, 4 or fewer inches. In some embodiments, a proximity may include a distance between user device 404 and wireless communication tag 408 of 4 or more inches. Wireless communication tag 408 may communicate physiology affecting information (PAI) 412 through one or more radio frequencies to user device 404. Physiologically modifying information 412 may include meal data, as described above. Meal data may include macronutrients of one or more food items, quantities of food items, micronutrients of one or more food items, and the like. Meal data may be pre-programmed into wireless communication tag 408 to include meal data associated with a meal approximate to wireless communication tag 408, such as a meal inside a lunchbox, a meal at a cafeteria, and the like. A user may write to wireless communication tag 408 to store any data as described throughout this disclosure in a memory of wireless communication tag 408, without limitation. In some embodiments, one or more manufacturing entities may program wireless communication tag 408 to include meal data of certain food items, such as, but not limited to, pre-made meals, snacks, frozen dinners, energy drinks, desserts, breakfasts, and the like. For instance, a manufacture may place wireless communication tag 408 on a side of a packaging of a breakfast sandwich. A user may scan wireless communication tag 408 whenever they consume the food item associated with wireless communication tag 408. In this example, wireless communication tag 408 may be passively powered which may reduce a cost of manufacture. In some embodiments, a user may program meal data into wireless communication tag 408 for each a specific meal. This process may be repeated indefinitely which may allow wireless communication tag 408 to be reusable. Wireless communication tag 408 may be programmed through a mobile application, NFC writer, RFID writer, and the like, without limitation.

Still referring to FIG. 4A, in some embodiments, user device 404 may be configured to activate bolus calculator 420 through GUI 416 as a function of physiology affecting information 412. Processor 424 of user device 404 may be programmed to be operable to run bolus calculator 420. In some embodiments, bolus calculator 420 may be stored in a memory of user device 404. Processor 424 may access a memory storing bolus calculator 420. Processor 424 may run bolus calculator 420. User device 404 may include and/or be similar to that of controller 204 as described above with reference to FIG. 2. The bolus calculator 420 may be operable to receive as an input, one or more of fats, carbohydrates, and/or proteins, and be operable to output a quantity of insulin to be delivered and/or rate of delivery of insulin for a user. In an embodiment, the bolus calculator 420 may be configured to calculate an insulin delivery locally, such as with one or more processors of user device 404. In other embodiments, the bolus calculator 420 may communicate with one or more external computing devices. One or more external computing devices may, for example, be operable to calculate an insulin delivery for a user and communicate the insulin delivery for the user to bolus calculator 420. A processor on the user device 404 may be operable to execute the bolus calculator 420 automatically once the user device 404 is within a certain proximity to wireless communication tag 408. In other embodiments, the user device 404 may generate one or more user prompts through GUI 416 requesting a user permission to run the bolus calculator 420. A user may confirm or reject the request for permission to execute the bolus calculator 420 via a user input, which may be via a touch or verbal input. The user device 404 may be operable to communicate with one or more medical devices, such as wearable medical device and wearable sensors described above with reference to earlier examples, without limitation.

In an example, the user device 404 may communication an insulin delivery calculated by bolus calculator 420 to one or more of the wearable medical devices, such as an insulin pump. For instance and without limitation, a user may be wearing a wearable injection device and approach wireless communication tag 408. The wireless communication tag 408 may transmit physiology affecting information 412, which may include meal data that is associated with a meal associated with the wireless communication device 408. For example, the wireless communication tag 408 may be positioned on a restaurant menu, a plate on which a meal is served, a box (such as a lunch box or Bento box) in which a meal is contained. A processor of the user device 404 may execute the bolus calculator 420 to determine a dosage of insulin to be delivered to compensate the user's blood glucose for the meal (e.g., carbohydrates, macronutrients and the like) indicated by the meal data within the physiology affecting information 412. Using the meal data of the physiology affecting information 412, the bolus calculator 420 is operable to calculate an amount of insulin and/or a rate of insulin delivery that compensates the user blood glucose for ingestion of the meal for the wearable injection device to delivery. In this example, the user device 404 may communicate a signal, for example, with the amount of insulin and/or a rate of insulin delivery embedded to the wearable injection device (shown in other examples). In situations where an entire meal is not consumed, the GUI 416 may be operable to include a slider that may be indicative of a portion size of one or more food elements of a meal, or the portion size of the entire meal. For example, the slider may include a horizontal bar with a left end indicative of a smallest portion (consumed) and a right end indicative of a largest portion (consumed). A user may interact with a slider, such as through swiping inputs, to select a portion size of a meal, before or after the meal has been consumed. Of course, other user interaction devices, such as radio buttons or the like may be used in place of the slider. Based on the user indication of the portion size consumed, the bolus calculator 420 may calculate and/or adjust an insulin delivery rate and/or quantity of insulin as a function of the portion size of a meal.

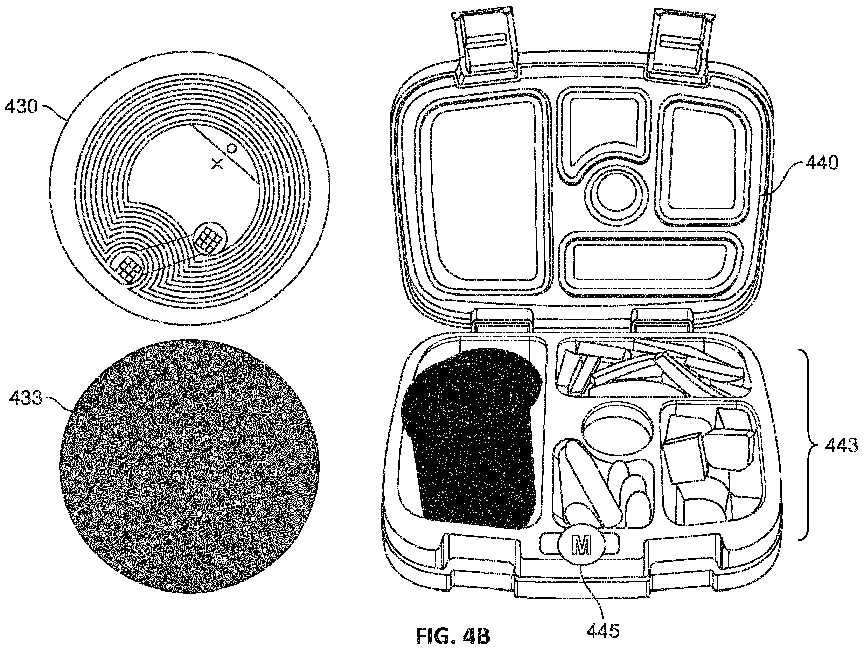

FIG. 4B illustrates an exemplary implementation of a wireless communication tag with meal container. In FIG. 4B, a meal container 440 may include a number of food items 443. A wireless communication tag, such as 430, may be coupled to an adhesive cover 433 that serves to both protect the wireless communication tag 430 and adhere it to a surface of an object, such as the meal container 440. The wireless communication tag 430 may have meal data stored on it. For example, a user or a user's guardian may have used a writing program on a user device, such as 204 of FIG. 2 or 404 of FIG. 4A, that is executed by the processor 424 to encode the wireless communication tag 430 with nutritional information (e.g., the number of grams of carbohydrates, protein and fat) of the respective food items 443. The meal data-encoded wireless communication tag 430 may be adhered to a location, such as 445, on the meal container 440.

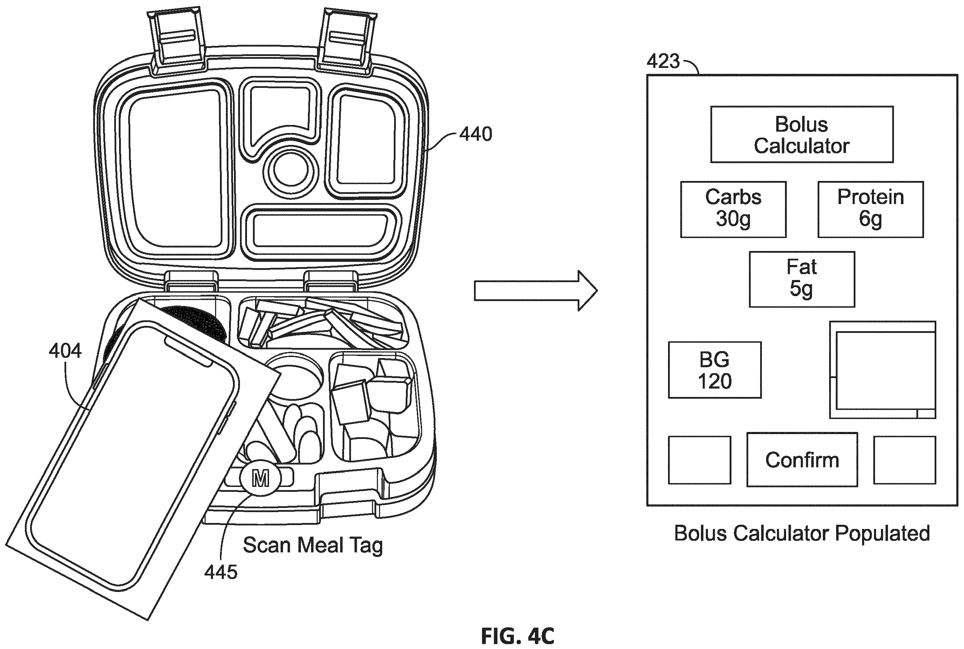

FIG. 4C illustrates an exemplary process utilizing the wireless communication tag and meal container shown in FIG. 4B. In an operational example, a user may place their user device 404 within reading range (e.g., 4-10 centimeters) of the meal tag 430 that is adhered at position 445. The user device 404 may read via a reader device, such as an NFC or a RFID reader (shown in other examples) the meal data encoded on the meal tag 430. The processor of the user device 404 may execute a bolus calculator, such as 420 shown in FIG. 4A, that takes the read meal data and calculates a bolus dosage and causes the presentation of information 423 on the user device GUI 416. The presented information may include the read meal data, such as 30 g of carbohydrates (carbs), 6 g of protein, 5 g of fat. In addition, the bolus calculator may obtain from the user device processor blood glucose measurement values. For example, the processor may have access to blood glucose measurement values delivered by a continuous blood glucose monitor or provided by an automated insulin delivery algorithm. The presented information 423 may include the user's blood glucose measurement value, e.g., 120. The bolus calculator may also provide a recommended bolus dosage, in this case 10 U, as part of the presented information 423. A prompt in the form of a confirm button with "Y?" or "N?" is provided for confirmation or rejection of the recommended bolus dosage.

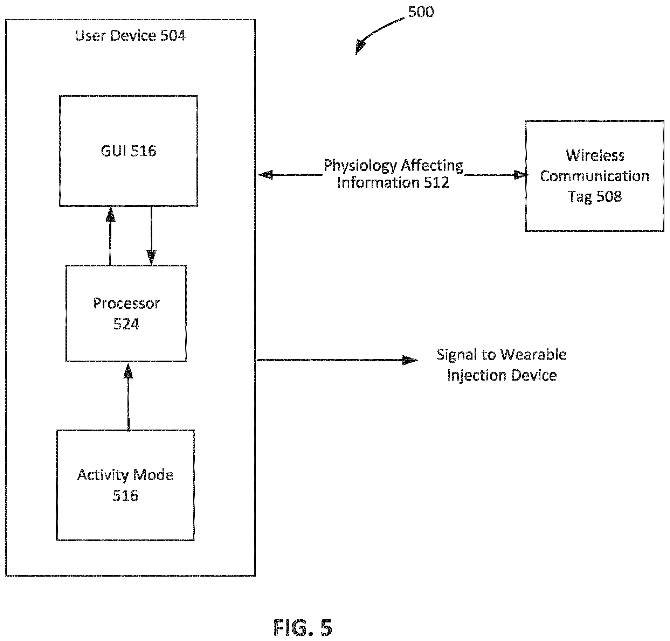

Referring now to FIG. 5, an exemplary embodiment of drug delivery system that utilizes physiology affecting information for indicating a user activity for a wearable medical device is presented. System 500 may include a user device 504, wireless communication tag 508, and physiology affecting information 512, as described above. The user device 504 may be similar to the user device 404 of FIG. 4A as well as controller 204 of FIG. 2 that were described in detail in earlier examples, so a detailed description is not reproduced. In the example of FIG. 5, the user device 504 may be placed in proximity to the wireless communication tag 508. A number of wireless communication tags 508 may be configured with physiology affecting information 512 that is related to a physical activity performed by the user. For example, a wireless communication tag 508 may be affixed to an exercise location, such as a fitness center, a particular space in the user's home where the user exercises, or the like. Additionally, or alternatively, a wireless communication tag 508 may be affixed to (or near) exercise equipment, such as, exercise shoes, entrances of gyms, barbells, dumbbells, cable machines, swimsuits, gym bags, swim goggles, a kayak, a paddleboard, ski equipment, and the like, without limitation. Wireless communication tag 508 may transmit exercise data, as described above, to user device 504 through physiology affecting information 512. Exercise data may include, without limitation, a type of exercise (e.g., aerobic or anaerobic, an expected exercise duration (e.g., a spin class typically lasts 50 minutes), an expected exercise intensity (e.g., this location is where a high intensity interval training class occurs), and the like. The user device 504 may be operable to generate one or more user prompts through GUI 516 to set a wearable medical device to activity mode 516. Alternatively, upon a receipt of the physiology affecting information 512, the user device 504 may cause activation of the activity mode 516. Activation of activity mode 516 may be performed through processor 524 of user device 504. Processor 524 of user device 504 may be programmed to be operable to activate activity mode 516. User device 504 may include and/or be similar to that of controller 204 as described above with reference to FIG. 1. The activity mode 516 may modify settings of an automated insulin delivery algorithm that may include adjusting one or more target setpoints of blood glucose levels, adjusting basal levels, modifying insulin delivery schedules (both basal and bolus dosages) and the like of a user by a wearable injection device. In some embodiments, a user confirms a selection of activity mode 516 for a wearable medical device through GUI 516.

Still referring to FIG. 5, user device 504 may generate one or more alerts for a user to consume one or more carbohydrates, proteins, and/or fats as a function of physiology affecting information 512. In a specific instance, physiology affecting information 512 may include the exercise data stored as the physiology affecting information 512 showing a basketball game of about 2 hours will start at 5:30 P.M. The user device 504 may present an alert, through GUI 516 and/or speakers (described with reference to other examples) of the user device 404, for the user to consume 20 grams of carbohydrates approximately 30 minutes before the basketball game. Alternatively, as another non-limiting example, user device 404 may use the physiology affecting information 512 that basketball shoes are in a gym bag to search a calendar to determine the user usually plays basketball at 5:30 P.M. and the current time is 2:45 P.M. In some embodiments, wireless communication tag 508 may include an internal clock, such as, but not limited to, a crystal oscillator. Wireless communication tag 508 may communicate a time generated by an internal clock of wireless communication tag 508 with user device 404. Activity mode 516 may include an increased frequency of sensing a user's blood glucose through a wearable medical device for a period of time. In some embodiments, a wearable medical device may enter activity mode 516 automatically as based on one or more detected heart rates, detected heart rate variabilities, and/or other physiological parameters of a user, which may be sensed on-board the wearable medical device and/or transmitted from user device 504. Wireless communication tag 508 and/or user device 504 may communicate with one or more other exercise monitoring devices, such as, but not limited to, heart rate monitors, blood oxygen sensors, temperature sensors, and the like. The wireless communication tag 508 may provide physiological affecting information 512 and the user device 504 may use the physiological affecting information 512 to produce data such as determining blood glucose variation during exercise, post exercise, and pre-exercise, which the user device 504 may to further improve insulin delivery of a wearable medical device. For instance, the wireless communication tag 508 may generate a timestamp of when user device 504 interacted with wireless communication tag 508 (or, alternatively the user device 504 can generate a time stamp when the physiology affecting information 512 is read from the wireless communication tag 508), which user device 504 may take a blood glucose reading of a user at or near the same time as the time of the timestamp. User device 504 may generate an exercise data pattern and/or trend for one or more users as a function of exercise data generated based on the physiology affecting information 512 and/or received from the wireless communication tag 508. The user device 504 may be operable to utilize an exercise data pattern to enhance insulin delivery through a wearable injection device (not shown in this example). For example, the user device 504 may send updated insulin delivery information in a signal to the wearable injection device.

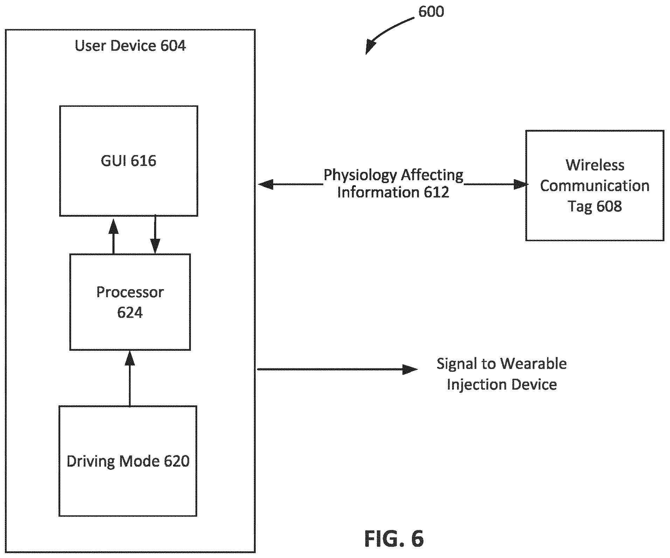

Referring now to FIG. 6, an exemplary embodiment of drug delivery system that utilizes physiology affecting information for indicating a driving condition of a user is presented. System 600 may include user device 604, wireless communication tag 608, and/or physiology affecting information 612, as described above. The user device 604 may be similar to the user device 404 of FIG. 4A as well as controller 204 of FIG. 2 that were described in detail in earlier examples, so a detailed description is not reproduced. In an example, the user device 604, such as a smartphone, may come within a proximity of wireless communication tag 608. The wireless communication tag 608 may be positioned in a vehicle, such as a truck, car, and the like, without limitation. In a non-limiting example, the wireless communication tag 608 may communicate physiology affecting information 612 with user device 404. User device 604 may be configured to initiate driving mode 620 as a function of physiology affecting information 612. User device 604 may initiate driving mode 620 through processor 624. Processor 624 may be programmed to be operable to activate and/or initiate driving mode 620. User device 604 may include and/or be similar to that of controller 204 as described above with reference to FIG. 2. Driving mode 620 may be an operational mode of an AID algorithm executed by the user device 604 that may include one or more of modifying one or more target setpoints of blood glucose of a user, modifying basal rates of insulin of a user, and the like of the AID algorithm. In some embodiments, driving mode 620 may include a wearable medical device increasing or decreasing a frequency of sensing of a user's blood glucose.

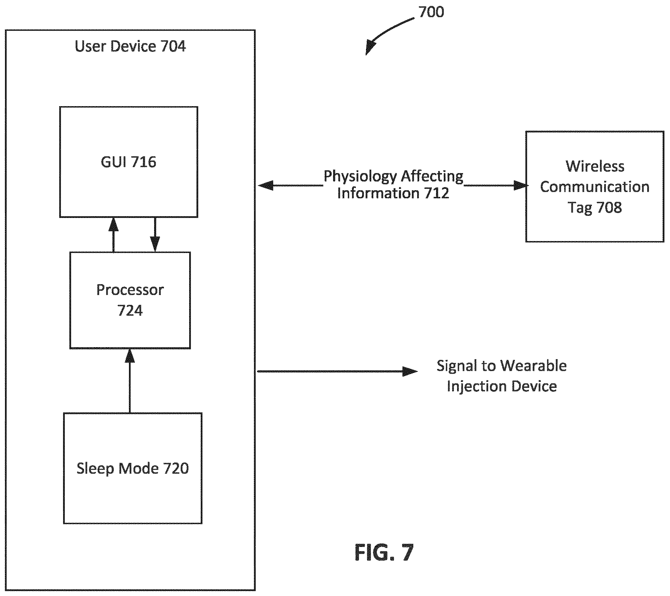

Referring now to FIG. 7, an exemplary embodiment of a drug delivery system that utilizes physiology affecting information for determining a user's sleep activity is presented. System 700 may include user device 704, wireless communication tag 708, and/or physiology affecting information 712 as described above. The user device 704 may be similar to the user device 404 of FIG. 4A as well as controller 204 of FIG. 2 that were described in detail in earlier examples, so a detailed description is not reproduced. In an example, the wireless communication tag 708 may be positioned by and/or near a sleeping location such as a bed, couch, and the like. Wireless communication tag 708 may communicate physiology affecting information 712, which may include sleep data, to user device 704 within a certain proximity. User device 704 may initiate sleep mode 720 be an operational mode of an AID algorithm executed by the user device 404 that is initiated as a function of physiology affecting information 710. When sleep mode 720 is initiated, a wearable medical device be requested to increase or decrease a frequency of sensing a user's blood glucose and the AID algorithm may be notified of the increased or decreased frequency of sensing.

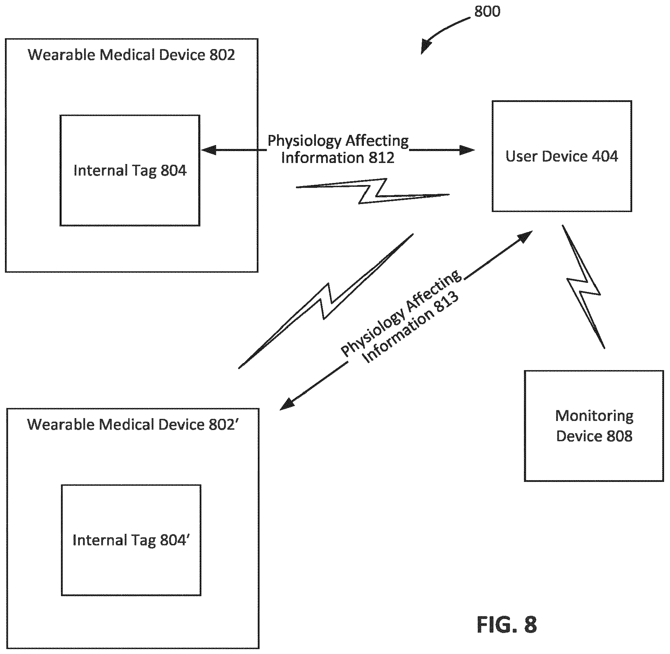

Referring now to FIG. 8, an exemplary embodiment of a system 800, particularly a communications network 800 of a wireless communication tag is presented. Communications network 800 may include wearable medical device 802. Wearable medical device 802 may include any wearable medical device as described throughout this disclosure, without limitation. Wearable medical device 802 may include an insulin pump, heart rate monitor, smart watch, and/or other device. In an example, wearable medical device 802 may include internal tag 804. An "internal tag" as used in this disclosure is a wireless communication tag housed within an object. Internal tag 804 may include one or more smart tags, RFID tags, NFC tags, and the like. For example, internal tag 804 may be a wireless communication tag, such as tag 300, as described above with reference to FIG. 3. Similarly, the user device 404 and/or the monitoring device 808 may be equipped with circuitry that is operational to read the physiology affecting information, such as 812 or 813 as well as other information from the internal tag, such as 804 and 804'. In an example, internal tag 804 may be housed within wearable medical device 802. In some embodiments, wearable medical device 802 may power internal tag 804. In other embodiments, internal tag 804 may include a passive power system, as described above.

With continued reference to FIG. 8, the system 800 may include monitoring device 808. A "monitoring device" as used in this example may be a computing device capable of continuous sensing of one or more individuals. The monitoring device 808 may include, without limitation, a heart rate monitor, EKG monitor, a respiratory monitor, and the like. In another example, the monitoring device 808 may include a continuous glucose monitoring (CGM) device as described in an earlier example. In some embodiments, monitoring device 808 may be configured to communicate with wearable medical device 802 through a wired and/or wireless connection. Monitoring device 808 may communicate through a low power Bluetooth connection (BLE), Wi-fi, and/or other connections. User device 404 may communicate with wearable medical device 802 and/or internal tag 804 of wearable medical device 802 through a wired and/or wireless connection. In some embodiments, internal tag 804 may communicate with user device 404 to switch from a first connectivity (e.g. first connectivity setting) to a second connectivity (e.g. second connectivity setting). Internal tag 804 may detect a proximity to user device 404 and communicate a signal prompting user device 404 to switch connectivity from wearable medical device 802 to monitoring device 808. User device 404 may detect a proximity to internal tag 804 and prompt a switch of connectivity based on the proximity of internal tag 804. As a non-limiting example, user device 404 may detect internal tag 804 and generate a GUI prompt asking a user if they want to switch connection between user device 404 and wearable medical device 802 (e.g. a first connectivity setting) to a connection between monitoring device 808 and user device 404 (e.g. a second connectivity setting). The user may confirm the prompt, which may cause user device 814 to cease communication with wearable medical device 802 and initiate communication with monitoring device 808. In another instance, user device 404 may be prompted to switch connectivity of a communication between monitoring device 808 and wearable medical device 802. Wearable medical device 802 may be in communication with monitoring device 808, such as, but not limited to, a Bluetooth and/or Wi-Fi communication. User device 404 may become within a proximity of internal tag 804. Internal tag 804 may communicate a prompt to user device 404 to switch a connectivity between wearable medical device 802 and monitoring device 808 (e.g. a first connectivity setting) to a connectivity of wearable medical device 802 to user device 404 (e.g. a second connectivity setting). A user may confirm the prompt, which may establish communications between wearable medical device 802 and user device 404 and wearable medical device 802 may cease communications with monitoring device 808. A first connectivity may include a wireless connection between user device 404 and/or monitoring device 808 and wearable medical device 802. A second connectivity may include a wireless communication between user device 404and/or wearable medical device 802 and monitoring device 808. Internal tag 804 may facilitate a transition of communication from a first connectivity to a second connectivity of user device 804, wearable medical device 802, and/or monitoring device 808. For instance, and without limitation, a user may switch a first wearable medical device 802 with a second wearable medical device 802'. A switch between the first wearable medical device 802 and the second wearable medical device 802' may cause a gap in data generated from the first wearable medical device 802 and/or monitoring device 808.

Still referring to FIG. 8, during a transition from the first wearable medical device 802 to the second wearable medical device 802', internal tag 804 may communicate with user device 404 to prompt user device 404 to switch from a first connectivity with user device 404 and wearable medical device 802 to a second connectivity of user device 404 and monitoring device 808. A transition from a first connectivity to a second connectivity may allow for a continuous generation of physiological data of a user between wearable medical device 802 and monitoring device 808. In some embodiments, wearable medical device 802 may communicate a deactivation of wearable medical device 802 to user device 404 through internal tag 804 and automatically switch a connectivity of user device 404 from communicating with wearable medical device 802 to communicating with monitoring device 808 and/or another wearable medical device 802' with internal tag 804' that is activated. In some embodiments, a proximity between the user device 404 and the internal tag 804 of the wearable medical device 802 which is to be swapped (e.g. a first wearable medical device 802), may cause a prompt on the user device 404 to switch the connectivity of the monitoring device 808 from a first connectivity between the monitoring device 808 and the wearable medical device 802 to a second connectivity between the monitoring device 808 and the user device 404. A proximity between the user device 404 and the internal tag 804' of the wearable medical device 802' by which the first wearable medial device 802 is swapped (e.g. a second wearable medical device), may cause a prompt on the user device 404 to switch the connectivity of the monitoring device 808 from the second connectivity between the monitoring device 808 and the user device 404 to a third connectivity between the monitoring device 808 and the wearable medical device 802'. In embodiments, a proximity between the user device 404 and the internal tag 804' of the wearable medical device 802' may cause a prompt on the user device 404 to switch the connectivity of the user device 404 from a connectivity between the user device 404 and the first wearable medical device 802 to a connectivity between the user device 404 and the second wearable medical device 802'. In a more detailed discussion of an operational example, the wearable medical device 802 may be a wearable injection device as described in earlier examples. A user may deactivate the wearable medical device 802 and may activate another wearable medical device 802' that is also a wearable injection device. Activation may occur in response to the user device 404 being placed in range to read to an internal tag 804' of the other wearable medical device 802'. In other embodiments, user device 404 may become within a proximity of internal tag 804. User device 404 may generate a prompt to switch between a first and/or second connectivity as a function of a communication of internal tag 804. In some embodiments, user device 404 may communicate with a cloud-computing network to update any physiological data generate from monitoring device 808.

Software related implementations of the techniques described herein may include, but are not limited to, firmware, application specific software, or any other type of computer readable instructions that may be executed by one or more processors. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

In addition, or alternatively, while the examples may have been described with reference to a closed loop algorithmic implementation, variations of the disclosed examples may be implemented to enable open loop use. The open loop implementations allow for use of different modalities of delivery of insulin such as smart pen, syringe or the like. For example, the disclosed AP application and algorithms may be operable to perform various functions related to open loop operations, such as the generation of prompts requesting the input of information such as weight or age. Similarly, a dosage amount of insulin may be received by the AP application or algorithm from a user via a user interface. Other open-loop actions may also be implemented by adjusting user settings or the like in an AP application or algorithm.

Some examples of the disclosed device may be implemented, for example, using a storage medium, a computer-readable medium, or an article of manufacture which may store an instruction or a set of instructions that, if executed by a machine (i.e., processor or microcontroller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magnetooptical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

Program aspects of the technology may be thought of as "products" or "articles of manufacture" typically in the form of executable code and/or associated data that is carried on or embodied in a type of non-transitory, machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

Although the present invention has been described above and is defined in the attached claims, it should be understood that the invention may alternatively be defined in accordance with the following embodiments:
1. A system, comprising:
   a user device; and
   at least one wireless communication tag, wherein the at least one wireless communication tag is configured to communicate a physiology affecting information to the user device, wherein the user device is configured to modify a mode of operation of a drug delivery device for delivery of a drug to the user as a function of the physiology affecting information.
2. The system of embodiment 1, wherein the drug delivery device is wearable.
3. The system of any one of the preceding embodiments, wherein the at least one wireless communication tag is further configured to communicate the physiology affecting information to the user device as a function of a proximity of the user device to the at least one wireless communication tag.
4. The system of any one of the preceding embodiments, wherein the physiology affecting information is generated by the wireless communication tag as a function of a location of the at least one wireless communication tag.
5. The system of any one of the preceding embodiments, wherein the user device is configured to:
   animate a graphical user interface (GUI) as a function of a proximity to the at least one wireless communication tag;
   receive user input through the GUI; and
   adjust a setting of the mode of operation of the drug delivery device as a function of the user input.
6. The system of any one of the preceding embodiments, wherein the user device is configured to generate an alert as a function of the physiology affecting information.
7. The system of any one of the preceding embodiments, wherein the physiology affecting information includes meal data, wherein the user device is configured to generate a bolus calculation for the drug delivery device as a function of the meal data.
8. The system of any one of the preceding embodiments, wherein modifying the mode of operation of the wearable medical device includes adjusting a basal setpoint of the drug delivery device.
9. The system of any one of the preceding embodiments, wherein the user device is configured to determine the mode of operation of the drug delivery device as a function of a historical trend of a plurality of modes of operation of the drug delivery device.
10. The system of any one of the preceding embodiments, wherein the drug delivery device includes an internal tag, wherein the internal tag is configured to wirelessly communicate with the user device.
11. The system of embodiment 9, wherein the internal tag is further configured to switch a connectivity of the drug delivery device from a first connectivity setting to a second connectivity setting.
12. A wireless communication tag usable with a drug delivery device, comprising:
   logic circuitry;
   a transmission element in communication with the logic circuitry; and
   a memory communicatively connected to the logic circuitry, wherein the memory contains instructions configuring the logic circuitry to:
   establish a data connection with at least a user device via the transmission element; and
   communicate a physiology affecting information to the user device through the data connection.
13. The wireless communication tag of embodiment 12, wherein the drug delivery device is wearable.
14. The wireless communication tag of any one of the embodiments 12 or 13, wherein logic circuitry is further configured to determine, through the transmission element, a proximity to the user device and establish the data connection as a function of the proximity.
15. The wireless communication tag of any one of the embodiments 12 to 14, wherein the wireless communication tag is further configured to establish a data connection with the drug delivery device.
16. The wireless communication tag of any one of the embodiments 12 to 15, wherein the logic circuitry is configured to receive meal data from an external computing device and communicate the physiology affecting information as a function of the meal data.
17. The wireless communication tag of any one of the embodiments 12 to 16, wherein the logic circuitry is configured to communicate the physiology affecting information as a function of a location of the wireless communication tag.
18. The wireless communication tag of any one of the embodiments 12 to 17, wherein logic circuitry is configured to enable the user device to generate a bolus calculation for a drug delivery device as a function of the physiology affecting information.
19. The wireless communication tag of any one of the embodiments 12 to 18, wherein the logic circuitry is configured to enable the user device to generate a graphical user interface (GUI) for at least a mode of operation of a drug delivery device as a function of the data connection.
20. The wireless communication tag of any one of the embodiments 12 to 19, wherein the wireless communication tag is passively powered.
21. The wireless communication tag of any one of the embodiments 12 to 20, wherein the logic circuitry comprises a processor.
22. The wireless communication tag of any one of the embodiments 12 to 21, wherein the wireless communication tag comprises a near field communication (NFC) tag.

## Claims

1. A system, comprising:
a user device; and
at least one wireless communication tag;
wherein the at least one wireless communication tag is configured to communicate a physiology affecting information to the user device,
wherein the user device is configured to modify a mode of operation of a drug delivery device for delivery of a drug to the user as a function of the physiology affecting information.

2. The system of claim 1, wherein the drug delivery device is a wearable medical device.

3. The system of any one of the preceding claims, wherein the at least one wireless communication tag is further configured to communicate the physiology affecting information to the user device as a function of a proximity of the user device to the at least one wireless communication tag.

4. The system of any one of the preceding claims, wherein the physiology affecting information is generated by the wireless communication tag as a function of a location of the at least one wireless communication tag.

5. The system of any one of the preceding claims, wherein the user device is configured to:
animate and/or generate a graphical user interface (GUI) as a function of a proximity to the at least one wireless communication tag;
receive user input through the GUI; and
adjust a setting of the mode of operation of the drug delivery device as a function of the user input.

6. The system of any one of the preceding claims, wherein the physiology affecting information includes meal data, wherein the user device is configured to generate a bolus calculation for the drug delivery device as a function of the meal data.

7. The system of any one of the preceding claims, wherein modifying the mode of operation of the drug delivery device includes adjusting a basal setpoint of the drug delivery device.

8. The system of claim 1, wherein the user device is configured to determine the mode of operation of the drug delivery device as a function of a historical trend of a plurality of modes of operation of the drug delivery device.

9. The system of any one of the preceding claims, if at least dependent on claim 2, wherein the drug delivery device includes an internal tag, wherein the internal tag is configured to wirelessly communicate with the user device, and wherein the internal tag is further configured to switch a connectivity of the drug delivery device from a first connectivity setting to a second connectivity setting.

10. A wireless communication tag usable with a drug delivery device, comprising:
logic circuitry;
a transmission element in communication with the logic circuitry; and
a memory communicatively connected to the logic circuitry, wherein the memory contains instructions configuring the logic circuitry to:
establish a data connection with at least a user device via the transmission element; and
communicate a physiology affecting information to the user device through the data connection.

11. The wireless communication tag of claim 10, wherein logic circuitry is further configured to determine, through the transmission element, a proximity to the user device and establish the data connection as a function of the proximity.

12. The wireless communication tag of any one of claims 10 or 11, wherein the wireless communication tag is further configured to establish a data connection with the drug delivery device.

13. The wireless communication tag of any one of claims 10 to 12, wherein the logic circuitry is configured to receive meal data from an external computing device and communicate the physiology affecting information as a function of the meal data.

14. The wireless communication tag of any one of claims 10 to 13, wherein the logic circuitry is configured to prompt the user device to generate one or more of:
a bolus calculation for a drug delivery device as a function of the physiology affecting information, and/or
a graphical user interface (GUI) for at least a mode of operation of a drug delivery device as a function of the data connection.

15. The wireless communication tag of any one of claims 10 to 14, wherein the wireless communication tag is passively powered, wherein the wireless communication tag comprises a near field communication (NFC) tag.
